(19) Europäisches Patentamt
European Patent Office
Office européen des brevets

(11) Publication number: 0 383 319 A2

# (12) EUROPEAN PATENT APPLICATION

(21) Application number: 90102950.4

(22) Date of filing: 15.02.90

(51) Int. Cl.5: C07D 471/04, A01N 43/90, //(C07D471/04,235:00,221:00)

(30) Priority: 17.02.89 JP 38996/89
16.01.90 JP 7970/90

(43) Date of publication of application:
22.08.90 Bulletin 90/34

(84) Designated Contracting States:
AT BE CH DE DK ES FR GB GR IT LI LU NL SE

(71) Applicant: TAKEDA CHEMICAL INDUSTRIES, LTD.
3-6, Doshomachi 2-chome Chuo-ku
Osaka(JP)

(72) Inventor: Ohta, Kazunari
31-14, 6 Zuiko 1-chome,
Higashi-yodogawa-ku,
JP-Osaka 533(JP)
Inventor: Ishida, Yasuo
A-410, 45 Yamadaminami, Suita,
JP-Osaka 565(JP)
Inventor: Yoshikawa, Harutoshi
304-103, 7 Otokoyama-sasatani, Yawata,
JP-Kyoto 614(JP)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

## (54) Imidazopyridines, their production and use.

(57) A compound of the formula (I):

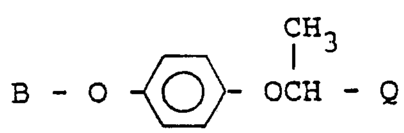

(I)

in which B is an imidazo[1,2-a]pyridin-2-yl group which may be substituted, Q is a cyano group or a group of the formula:

$$-\overset{\overset{\displaystyle O}{\|}}{C}-A-R$$

wherein A is an oxygen or sulfur atom or a group of the formula :-NR¹- (wherein R¹ is a hydrogen atom or a lower alkyl group), R is a hydrogen atom, a hydrocarbon group which may be substituted or a heterocyclic group which may be substituted, or its salt, which is useful as selective herbicide, and its production and use.

EP 0 383 319 A2

# Imidazopyridines, Their Production and Use

## BACKGROUND OF THE INVENTION

### 1. Field of the Invention

This invention relates to imidazopyridines, their production and use. The imidazopyridines of the invention are novel and possess excellent herbicidal effects against weeds in paddy field and upland field, and do not damage crops such as rice, wheat, barely, soybean, etc., and accordingly are useful as excellent selective herbicides.

### 2. Description of the Prior Arts

Many kinds of herbicides have been developed and actually used, but they are not sufficient in the view points of herbicidal effect against weeds, damage on the crops, toxicity against mammals, fishes and shellfishes, environmental pollution, and the like, and accordingly development of an improved herbicide with high selectivity has been earnestly desired.

The inventors of this invention have studied to develop selective herbicides possessing superior herbicidal effect and no damage against crops, and found that the compounds of the following formula (I) and their salts (especially, their optical isomers) possess a potent herbicidal activity and show remarkably reduced damage against crops such as rice, wheat, barley, soybean, etc., and that they are useful as herbicides with high selectivity, and finally completed the invention through further intensive study based on the above-mentioned facts.

## SUMMARY OF THE INVENTION

The present invention relates to a compound of the formula (I):

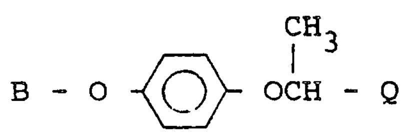

(I)

in which B is an imidazo[1,2-a]pyridin-2-yl group which may be substituted, Q is a cyano group or a group of the formula:

$$-\overset{\overset{O}{\|}}{C}-A-R$$

wherein A is an oxygen or sulfur atom or a group of the formula :-$NR^1$- (wherein $R^1$ is a hydrogen atom or a lower alkyl group), R is a hydrogen atom, a hydrocarbon group which may be substituted or a heterocyclic group which may be substituted; when A is -$NR^1$-, R and $R^1$ may combine to form a heterocyclic group together with the adjacent nitrogen atom; or when A is an oxygen atom or -$NR^1$-, R may be a group of the formula:

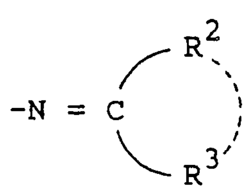

wherein $R^2$ and $R^3$ each are a lower alkyl, lower alkoxy or lower alkylthio group, or $R^2$ and $R^3$ may form a 5-

or 6-membered ring together with the adjacent carbon atom, or a salt thereof.

Further, the present invention relates to a process for preparing a compound of the formula (I) or its salt, which comprises reacting a compound of the formula (II):

B - Z (II)

wherein Z is a removable group, and B is as defined in the above,
or its salt with a compound of the formula (III):

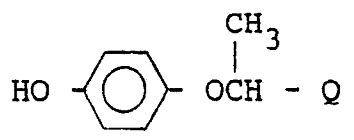
(III)

wherein Q is as defined in the above,
in the presence of a base.

The present invention also relates to a process for preparing a compound of the formula (I) or its salt which comprises reacting a compound of the formula (IV):

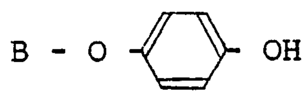
(IV)

wherein B is as defined in the above,
or its salt with a compound of the formula (V):

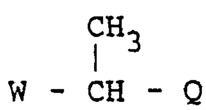
(V)

wherein W is a removable group, and Q is as defined in the above,
in the presence of a base.

Also, it provides a herbicidal composition comprising a compound of the formula (I) or its salt.

## PREFERRED EMBODIMENTS OF THE INVENTION

In the formula (I), B is an imidazo[1,2-a] pyridin-2-yl group which may be substituted, and the substituent(s) on the pyridine ring (hereinafter shown as Xn and n means 1 or 2, and when n is 2, the two substituents X may be, the same or different and independently), may be a halogen atom, nitro group, a lower alkyl group which may be substituted by halogen atom(s). The halogen atom for X may be fluorine, chlorine, bromine, iodine, etc. The lower alkyl moiety in the lower alkyl group which may be substituted by halogen atom(s) may be a straight or branched chain $C_{1-6}$ alkyl group such as methyl, ethyl, n-propyl, iso-propyl, n-butyl, t-butyl, etc. The halogen moiety in the lower alkyl group which may be substituted by one to three of halogen atom(s) may be fluorine, chlorine, bromine, etc.

Preferable examples of the substituents for X are a halogen atom and a lower alkyl group which may be substituted with halogen atom(s), and the more preferable examples are chlorine, bromine, methyl and trifluoromethyl.

The substituent on the imidazole ring (hereinafter shown as Y) may be, for example, a halogen atom, cyano group, nitro group, an acyl group, a lower alkoxycarbonyl group, a carbamoyl group which may be substituted by a lower alkyl group, carboxyl group, a lower alkylthio group, a lower alkylsulfinyl group, a lower alkylsulfonyl group, a lower alkylamino, a di-lower alkylamino, a lower alkoxy, a lower alkylsulfamoyl, a di-lower alkylsulfamoyl, a lower alkyl group which may be substituted by one to four of a halogen, hydroxyl, amino, a lower alkylamino, a di- lower alkylamino, a lower alkoxy and a lower alkylthio, and the like. The halogen atom for Y may be fluorine, chlorine, bromine, iodine, etc. The acyl group for Y may be $C_{1-7}$ acyl groups derived from aliphatic carboxylic acids such as formic acid, acetic acid, n-propionic acid, n-butyric acid or aromatic carboxylic acids such as benzoic acid, for example, formyl, acetyl, propionyl,

butyryl, benzoyl, and the like. The lower alkoxycarbonyl groups for Y may be the ones having 2 - 6 carbon atoms, for example, methoxycarbonyl, ethoxycarbonyl, n-propoxy- carbonyl, iso-propoxycarbonyl, n-butoxycarbonyl, iso-butoxycarbonyl, sec-butoxycarbonyl, tert-butoxycarbonyl, and the like.

The carbamoyl group which may be substituted by lower alkyl group(s) for Y may be unsubstituted carbamoyl group or a mono- or di-lower alkylcarbamoyl group substituted by a lower alkyl having 1 to 6 carbon atoms, for example, carbamoyl, methylcarbamoyl, dimethylcarbamoyl, methylethylcarbamoyl, and the like.

The lower alkylthio group for Y may be a straight or branched chain $C_{1-6}$ alkylthio group such as methylthio, ethylthio, n-propylthio, iso-propylthio, n-butylthio, iso-butylthio, sec-butylthio, tert-butylthio, n-pentylthio, n-hexylthio, and the like.

The lower alkylsulfinyl group for Y may be a straight or branched chain $C_{1-6}$ alkylsulfinyl group such as methylsulfinyl, ethylsulfinyl, n-propylsulfinyl, n-hexylsulfinyl, and the like.

The lower alkylsulfonyl group for Y may be a straight or branched chain $C_{1-6}$ lower alkylsulfonyl group such as methylsulfonyl, ethylsulfonyl, n-propylsulfonyl, iso-propylsulfonyl, n-butylsulfonyl, and the like.

The lower alkylamino group for Y may be a straight or branched chain $C_{1-6}$ alkylamino group such as methylamino, ethylamino, n-propylamino, iso-propylamino, and the like.

The di-lower alkylamino group for Y may be a di-straight or branched chain $C_{1-6}$ alkylamino group such as dimethylamino, diethylamino, methylethylamino, and the like.

The lower alkoxy group for Y may be a straight or branched chain $C_{1-6}$ alkoxy group such as methoxy, ethoxy, n-propoxy, iso-propoxy, and the like.

The lower alkylsulfamoyl group for Y may be a straight or branched chain $C_{1-6}$ alkylsulfamoyl group such as methylsulfamoyl, ethylsulfamoyl, n-propylsulfamoyl, and the like.

The di-lower alkylsulfamoyl group for Y may be a di-straight or branched chain $C_{1-6}$ alkylsulfamoyl group such as dimethylsufamoyl, diethylsulfamoyl, methylethylsulfamoyl, and the like.

The lower alkyl moiety in the lower alkyl group which may be substituted by one to four of a halogen, hydroxy, amino, lower alkylamino, di-lower alkylamino, lower alkoxy and lower alkylthio with respect to Y may be a straight or branched chain $C_{1-6}$ alkyl group such as methyl, ethyl, n-propyl, iso-propyl, n-butyl, tert-butyl, and the like. The halogen atom as the substituent may be fluorine, chlorine, bromine, iodine, etc., the lower alkylamino group may be a straight or branched chain $C_{1-6}$ alkylamino group such as methylamino, ethylamino, n-propylamino, iso-propylamino, etc., the di-lower alkylamino group may be a di-straight or branched chain $C_{1-6}$ alkylamino group such as dimethylamino, diethylamino, methylethylamino, etc., the lower alkoxy group may be a straight or branched chain $C_{1-6}$ alkoxy group such as methoxy, ethoxy, n-propoxy, iso-propoxy, etc., and the lower alkylthio group may be a straight or branched chain $C_{1-6}$ alkylthio group such as methylthio, ethylthio, n-propylthio, iso-propylthio, etc.

Preferable substituents shown by Y may be a halogen, cyano, nitro, a $C_{1-4}$ acyl, a $C_{1-4}$ alkoxycarbonyl, carbamoyl, a $C_{1-4}$ alkylsulfonyl and the like.

The suitable halogen atoms used as the substituent shown by Y may be chlorine and bromine, the suitable $C_{1-4}$ acyl groups may be formyl and acetyl, the suitable $C_{1-4}$ alkoxycarbonyl groups may be methoxycarbonyl, ethoxycarbonyl, etc., and the suitable $C_{1-4}$ alkylsulfonyl groups may be methylsulfonyl, ethylsulfonyl, etc. More preferable substituents shown by Y may be cyano, nitro, and the like.

Q is a cyano group or a group of the formula:

$-\overset{\overset{O}{\|}}{C}-A-R$, in which A is an oxygen or sulfur atom or a group of $-NR^1-$ ($R^1$ is a hydrogen atom or a lower alkyl group as exemplified in the lower alkyl group for X).

The hydrocarbon groups for R include an alkyl, aryl, aralkyl, alkenyl and alkynyl groups. These groups may possess one to five substituents. The alkyl group may be the lower alkyl group as exemplified for X in the above or a $C_{7-10}$ alkyl group such as heptyl group, octyl, etc. These alkyl groups may be substituted by a halogen, hydroxy, amino, mercapto, nitro, a lower alkylamino, a di-lower alkylamino, a lower alkylthio, a lower alkylsulfinyl, a lower alkylsulfonyl, a lower alkoxy, a tri-lower alkylsilyl, a lower alkoxycarbonyl or a group of the formula:

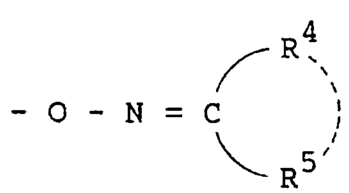

wherein $R^4$ and $R^5$ are hydrogen atom or have, the same or different, the same meanings as above $R^2$ and $R^3$.

Among the substituents on the above alkyl group, the halogen atom may be fluorine, chlorine, bromine, iodine, etc., the lower alkylamino group may be a straight or branched chain $C_{1-6}$ lower alkylamino group such as methylamino, ethylamino, n-propylamino, isopropylamino, etc., the di-lower alkylamino group may be a straight or branched chain $C_{1-6}$ di-lower alkylamino group such as dimethylamino, diethylamino, methylethylamino, etc., the lower alkylthio group may be a straight or branched chain $C_{1-6}$ lower alkylthio group such as methylthio, ethylthio, n-propylthio, iso-propylthio, etc., the lower alkylsulfinyl group may be a straight or branched chain $C_{1-6}$ lower alkylsulfinyl group such as methylsulfinyl, ethylsulfinyl, n-propylsulfinyl, n-hexylsulfinyl, etc., the lower alkylsulfonyl group may be a straight or branched chain $C_{1-6}$ lower alkylsulfonyl group such as methylsulfonyl, ethysulfonyl, n-propylsulfonyl, iso-propylsulfonyl, n-butylsulfonyl, etc., the lower alkoxy group may be a straight or branched chain $C_{1-6}$ lower alkoxy group such as methoxy, ethoxy, n-propoxy, isopropoxy, etc., the tri-lower alkylsilyl group may be a straight or branched chain $C_{1-6}$ tri-lower alkylsilyl group such as trimethylsilyl, tert-butyldimethylsilyl, etc., and the lower alkoxycarbonyl group may be a $C_{2-6}$ alkoxycarbonyl group such as methoxycarbonyl or ethoxycarbonyl. The lower alkyl group for $R^2$, $R^3$, $R^4$ and $R^5$ in the above formulae:

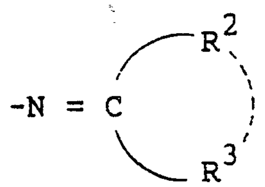 , 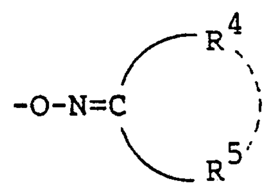

may be a straight or branched chain $C_{1-6}$ alkyl or cyclic $C_{3-6}$ alkyl group such as methyl, ethyl, n-propyl, iso-propyl, n-butyl, tert-butyl, cyclopropyl, cyclo- hexyl, etc., the lower alkoxy group for $R^2$, $R^3$, $R^4$ and $R^5$ may be a straight or branched chain $C_{1-6}$ alkoxy group such as methoxy, ethoxy, n-propoxy, iso-propoxy, etc., and the lower alkylthio group for $R^2$, $R^3$, $R^4$ and $R^5$ may be a straight or branched chain $C_{1-6}$ alkylthio group such as methylthio, ethylthio, n-propylthio, iso-propylthio, etc. And further, $R^2$ and $R^3$ or $R^4$ and $R^5$ may combine to form a 5- or 6-membered ring such as $C_{5-6}$ cycloalkane (e.g., cyclopentane, cyclohexane), together with the adjacent carbon atom. Further, $R^4$ and $R^5$ may be hydrogen atom.

Among the aryl, heterocyclic or aralkyl group which may be substituted with respect to R, the aryl group may be a $C_{6-14}$ aryl group such as phenyl, naphtyl, biphenyl, etc., the heterocyclic group may be a 5- or 6-membered ring containing 1 or 2 hetero atoms such as nitrogen atom, oxygen atom, sulfur atom or condensed ring thereof such as pyrrolyl, thienyl, furyl, pyrazolyl, imidazolyl, thiazolyl, oxazolyl, pyridyl, pyridazinyl, pyrimidinyl, pyrazinyl, quinolyl, etc., and the aralkyl group may be a $C_{7-19}$ aralkyl group such as benzyl, phenethyl, phenylpropyl, biphenylmethyl, trityl, etc.

The substituent on these aryl group, heterocyclic group and aralkyl group may be, for example, a lower alkyl group, a halogen atom, nitro group, cyano group, a lower alkoxy group, a lower alkoxycarbonyl group, and the like.

Among the above substituents on the aryl group, heterocyclic group and aralkyl group, the lower alkyl group may be a straight or branched chain $C_{1-6}$ alkyl group such as methyl, ethyl, n-propyl, iso-propyl, etc., thc halogen atom may be fluorine, chlorine, bromine, iodine, etc., the lower alkoxy group may be a straight or branched chain $C_{1-6}$ alkoxy group such as methoxy, ethoxy, n-propoxy, iso-propoxy, etc., the lower alkoxycarbonyl group may be a $C_{1-6}$ alkoxycarbonyl group such as methoxycarbonyl, ethoxycarbonyl, n-propoxycarbonyl, iso-propoxycarbonyl, etc.

The suitable alkenyl group for R may be $C_{2-6}$ alkenyl such as allyl, iso-propenyl, 1-butenyl, 2-pentenyl, 2-hexenyl, etc.

The suitable alkynyl group for R may be $C_{2-6}$ alkynyl group such as propargyl, 2-butynyl, 3-butynyl, 3-pentynyl, 3-hexynyl, etc.

The substituents on these alkenyl group and alkynyl group may be the same ones as exemplified for the above-mentioned alkyl group.

The heterocyclic group as formed by R and $R^1$ together with the adjacent nitrogen atom may be a 5- or 6-membered nitrogen-containing heterocyclic group such as pyrrolidyl, piperidyl, morpholyl or isooxazolyl.

When A is an oxygen atom or $-NR^1-$, R may be a group of the formula:

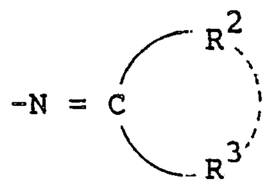

wherein $R^2$ and $R^3$ are a lower alkyl group, a lower alkoxy group or a lower alkylthio group, or $R^2$ and $R^3$ may combine to form a 5- or 6-membered ring together with the adjacent carbon atom.

The suitable substituent shown by Q is a group of the formula:

$-\overset{O}{\overset{\|}{C}}-A-R$. In the group of the formula:

$-\overset{O}{\overset{\|}{C}}-A-R$, the lower alkyl group for $R^1$ when A is $-NR^1-$ is preferably methyl or the like.

The suitable alkyl groups for R may be a straight or branched chain $C_{1-6}$ alkyl such as methyl, ethyl, n-propyl, iso-propyl, n-butyl, or a cyclic $C_{3-6}$ alkyl such as cyclohexyl, etc. These alkyl groups may possess one to three substituents, such as the above-mentioned halogen, hydroxy, mercapto, lower alkylamino, di-lower alkylamino, lower alkylthio, lower alkoxy, tri-lower alkylsilyl, lower alkoxycarbonyl or a group of the formula:

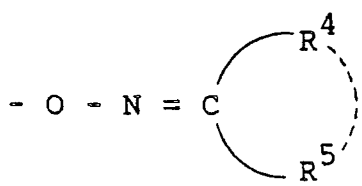

wherein $R^4$ and $R^5$ are as defined above. More suitable substituents on the above alkyl group are chlorine, trimethylsilyl or a group of the formula:

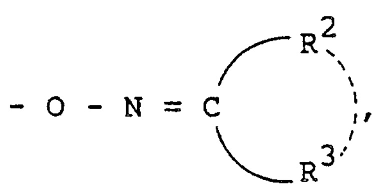

wherein $R^2$ and $R^3$ are preferably a lower alkyl, more preferably methyl.

And further, the suitable examples of R is the above-mentioned aryl group which may be substituted by one to five of a lower alkyl, a halogen, nitro, cyano, a lower alkoxy and a lower alkoxycarbonyl, or the above-mentioned lower alkenyl or lower alkynyl group.

More suitably, R is a phenyl group which may be substituted by one to five of a lower alkyl, a halogen, nitro and a lower alkoxy, or allyl or propargyl.

Interesting groups of the compounds (I) and salt thereof are a compound of the formula:

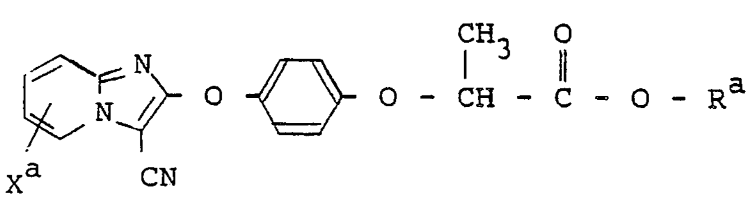

($I^a$)

wherein $X^a$ is a halogen atom, and $R^a$ is a $C_{1-4}$ alkyl or $C_{2-4}$ alkenyl group,
or its salt;
a compound of the formula:

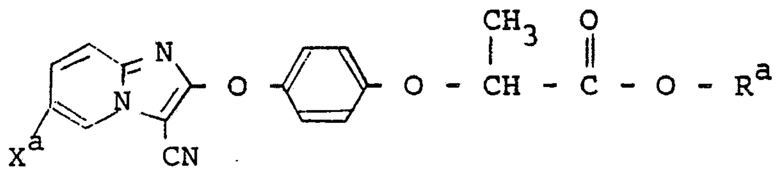

($I^b$)

wherein the symbols have the same meanings as defined above,
or its salt; and
a compound of the formula:

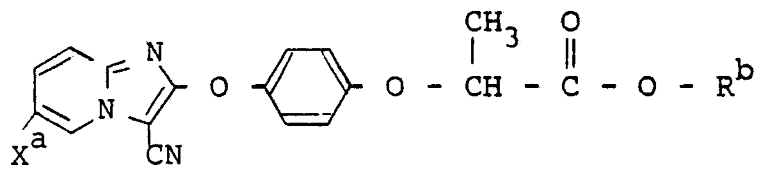

($I^c$)

wherein $X^a$ has the same meaning as defined above and $R^b$ is a $C_{2-4}$ alkenyl group,
or its salt.

In the above formulae ($I^a$), ($I^b$) and ($I^c$), the halogen for $X^a$ may be chlorine, bromine or fluorine; the $C_{1-4}$ alkyl for $R^a$ may be methyl, ethyl, n-propyl, i-propyl, n-butyl, i-butyl or t-butyl, preferably a $C_3$ or $C_4$ alkyl such as n-propyl, i-propyl or n-butyl; and the $C_{2-4}$ alkenyl for $R^a$ and $R^b$ may be vinyl, allyl, iso-propenyl or 1-butenyl, preferably allyl.

The salts of the compounds (I) [inclusive of ($I^a$), ($I^b$) and ($I^c$)] include the acid addition salt with an inorganic acid or organic acid or the base salt with an inorganic or organic base. The inorganic acid which can form the inorganic acid addition salt may be hydrochloric acid, hydrobromic acid, sulfuric acid, nitric acid, phosphoric acid, etc., and the organic acid which can form the organic acid addition salt may be p-toluenesulfonic acid, methanesulfonic acid, formic acid, trifluoroacetic acid, etc. The inorganic base salt may be the salt with an alkali metal such as sodium, potassium, etc., an alkali earth metal such as calcium, etc., ammonia or the like, and the organic base salt may be the salt with a base such as dimethylamine, triethylamine, piperazine, pyrrolidine, piperidine, benzylamine, etc.

The compounds (I) or their salts of this invention possess an asymmetric carbon atom, and accordingly may exist as two kinds of optical isomers. This invention includes d- and l-isomers of the compounds (I) or their salts znd also their mixtures in an optional ratio.

The compounds (I) and their salts (including the optical isomers, hereinafter) possess excellent herbi cidal action with very small amount against Poaceae weeds in paddy field and upland field (e.g., ), and also possess high safety with less damage against not only broad leaf crops (e.g., soybean, cotton plant, sugar beet, rape plant or sunflower), but also Poaceae crops (e.g., rice).

The compounds (I) and their salts exert effect as herbicide with superior selectivity between the crops and various kinds of weeds, possess low toxicity against mammals, fishes and shellfishes, do not pollute environment, and accordingly can be safely used as herbicides in paddy field, upland field, orchard and wild field. Also, in one aspect, they can be used of an increased production of broad leaf crops or Poacease crops (especially soybean).

The herbicidal composition containing the compound (I) and its salt may be in any of the conventional forms of agricultural chemicals. That is, one or more kinds of the compounds (I), or their salts (especially their optical isomers) are dissolved or dispersed in a proper liquid carrier, or mixed with or absorbed on a proper solid carrier in accordance with a use purpose to give a formulation such as emulsion, oily formulation, spray, water-miscible formulation, powder, DL-type powder, granule, fine granule, fine granule F, tablet, etc. These formulations can be prepared by a known method by adding emulsifying agent, dispersing agent, spreader, penetrating agent, wetting agent, mucilage, stabilizer, etc., if necessary.

The liquid carriers (solvents) to be used may be a solvent, for example, water, alcohols (e.g., methanol, ethanol, n-propanol, iso-propanol, ethyleneglycol, etc.), ketones (e.g., acetone, methyl ethyl ketone, etc.), ethers (e.g., dioxane, tetrahydrofuran, ethyleneglycol monomethyl ether, diethyleneglycol monomethyl ether, propyleneglycol monomethyl ether, etc.), aliphatic hydrocarbons (e.g., kerosene, paraffin oil, fuel oil, machine oil, etc.), halogenated hydrocarbons (e.g., dichloromethane, chloroform, tetrachlorocarbon, etc.), acid amides (e.g., dimethylformamide, dimethylacetamide, etc.), esters (e.g., ethyl acetate, butyl acetate, aliphatic glycerin ester, et.c), nitriles (e.g., acetonitrile, propionitrile, etc.), and these solvents may be used

alone or in an optional mixture thereof.

The suitable solid carriers (diluent, filler) may be vegetable powders (e.g., soybean powder, tobacco powder, wheat powder, wood powder, etc.), mineral powders (e.g., clay such as kaolin, bentonite, Japanese acid clay, etc., talc such as talc powder, roseki powder, etc., silica such as diatomaceous earth, mica powder, etc.), alumina, sulphur powder, active charcoal, and these carrier may be used alone or in an optional mixture thereof.

Surface active agents to be used as the emulsifying agent, spreader, penetrating agent, dispersing agent, etc., may be non-ionic or anionic surfactants such as soaps, polyxyethylenealkylarylethers [e.g., Neugen, E.A 142® (manufactured by Dai-Ichi Kogyo Seiyaku Co., Ltd.)], polyoxyethylenearylesters [e.g., Nonal® (manufactured by Toho Chemical Co., Ltd.)], alkyl sulfates [e.g., Emal 10®, Emal 40® (manufactured by Kao Corporation)], alkyl sulfonates [e.g., Neogen®, Noegen T® (manufactured by Dai-Ichi Kogyo Seiyaku Co., Ltd.): Neopelex® (manufactured by Kao Corporation)], polyethyleneglycol ethers [e.g., Nonipole 85®, Nonipole 100®, Nonipole 160® (manufactured by Sanyo Chemical Industries, Ltd.)], polyhydric alcohol esters [e.g., Tween 20®, Tween 80® (manufactured by Kao Corporation)].

Suitable content of the compound (I) or its salt contained in a herbicidal composition may be about 1 to 90 weight % in case of emulsion, water miscible formulation, etc., or about 0.01 to 10 weight % in case of oily formulation, powder, DL-type powder, etc., or about 0.05 to 10 weight % in case of fine granule, granule, etc. However, the content may be increased or decreased in accordance with the use purpose. Emulsion, wettable powders, etc., are sprayed after diluting with water or the like to, for instance, 100 to 100,000 times of volume.

The amount of the compound (I) or its salt to be used as a herbicide depends on place, time, method, weed, crops, etc., to be applied, and generally about 0.25 to 50g, more preferably about 1 to 10g of the active ingredient [the compound (I) or its salt] may be used for one are of paddy field, and about 0.25 to 20g, more preferably about 1 to 10g for one are of farm.

The compound (I) or its salt may be applied to soil before growth of undesirable weeds (application before germination), or may be applied to a part of growing weed upper than soil (application after germination).

The herbicide of this invention may be used in combination with any other kinds of herbicides, plant growth regulators, germicidal agents (e.g., organic chloric germicidal agents, organic sulphuric germicidal agents, azole germicidal agents, antimicrobial agents, etc.), insecticides (e.g., pyrethroid insecticides, organic phosphoric insecticides, carbamate insecti cides, etc.), tick killing agents, nematocides, synergists, enticing agents, evading agents, coloring agents, fertilizers, and the like.

The compound (I) of this invention or its salt can be prepared from an optionally substituted imidazo-[1,2-a]pyridine compound having a removable group at the 2nd position of the formula (II):

B - Z (II)

wherein Z is a removable group (e.g., a halogen atom such as fluorine, chlorine, bromine or iodine, a $C_{6-10}$ aromatic hydrocarbon sulfonyloxy such as p-toluenesulfonyloxy, or a $C_{1-6}$ alkanesulfonyloxy such as methanesulfonyloxy), and B is the same meaning as defined in the above formula (I), or its salt.

The imidazo[1,2-a]pyridine compound (II) having a removable group at the 2nd position or its salt can be derived to the compound (I) or its salt of this invention through the following 5 routes shown as A, B, C, D and E.

Route A:

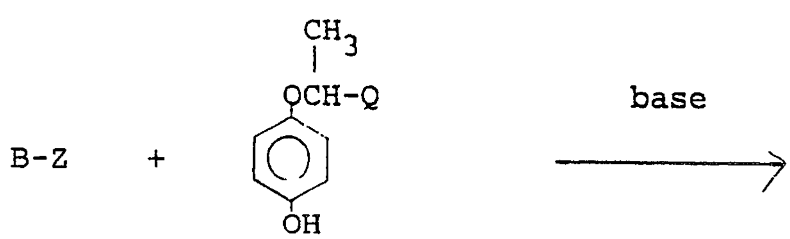


(II) (III)

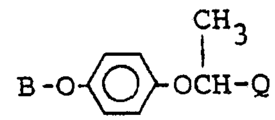

(I)

According to the Route A, the compound (I) or its salt of this invention can be directly prepared by reacting an imidazo[1,2-a]pyridine compound (II) having a removable group at the 2nd position or its salt, with a 2-(4-hydroxyphenoxy) propionic acid derivative (III) in the presence of a base. The derivatives (III) themselves are known compounds and can be prepared by a conventional method.

The bases to be used in the reaction may be, for example, inorganic bases (e.g., alkali metal hydroxide or carbonate such as sodium hydroxide, potassium hydroxide or sodium carbonate, or alkali metal hydride such as sodium hydride).

The reaction is preferably conducted to an inert solvent, for example, aromatic hydrocarbons such as benzene, toluene, etc., halogenated hydrocarbons such as dichloromethane, chloroform, carbon tetrachloride, etc., ethers such as diethyl ether, diisopropyl ether, dioxane, tetrahydrofuran (hereinafter abbreviated as THF), etc., nitriles such as acetonitrile, etc., ketones such as acetone, methyl ethyl ketone, etc., esters such as ethyl acetate, butyl acetate, etc., dimethylformamide, dimethyl sulfoxide, or the like.

The reaction temperature is usually about 20 to 200 °C, preferably 80 to 150 °C. The reaction is completed in about 30 minutes to 20 hours, and the completion can be confirmed by thin layer chromatography or high pressure liquid chromatography.

<u>Route B:</u>

The reaction can be summarized by the following scheme.

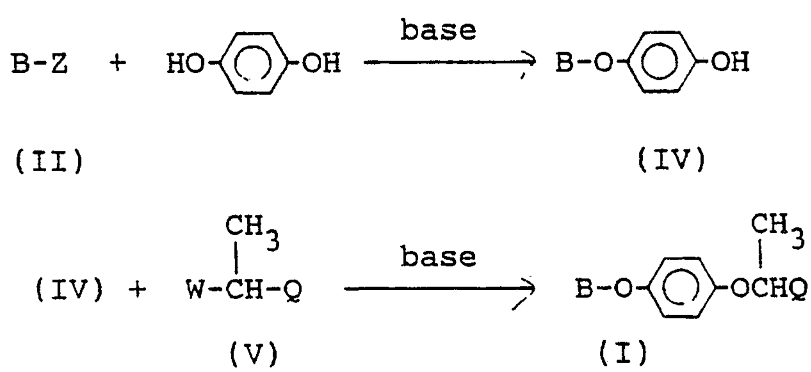


In the above Route B, Z has the same meaning as above, and W means a removable group (for example, halogen atom such as fluorine, chlorine, bromine or iodine, $C_{6-10}$ aromatic hydrocarbon-sulfonyloxy such as p-toluenesulfonyloxy or $C_{1-6}$ alkane-sulfonyloxy such as methanesulfonyloxy), and Z and W may be the same or different.

According to the Route B, the compound (IV) or its salt can be prepared by reacting an imidazo[1,2-a]-pyridine compound (II) or its salt with hydroquinone in the presence of a base.

The bases to be used in the reaction may be, for example, inorganic bases (e.g., alkali metal hydroxide or carbonate such as sodium hydroxide, potassium hydroxide, sodium carbonate, or alkali metal hydride such as sodium hydride). The reaction is preferably conducted in an inert solvent, for example, aromatic hydrocarbons such as benzene, toluene, etc., halogenated hydrocarbons such as dichloromethane, chloroform, carbon tetrachloride, etc., ethers such as diethyl ether, diisopropyl ether, dioxane, THF, etc., nitriles such as acetonitrile, etc., ketones such as acetone, methyl ethyl ketone, etc., esters such as ethyl acetate, butyl acetate, etc., dimethylformamide, dimethylsulfoxide, or the like.

The reaction temperature is usually about 20 to 200 °C, preferably 80 to 150 °C. The reaction is completed in about 30 minutes to 20 hours, and the completion can be confirmed by thin layer chromatography or high pressure liquid chromatography.

The desired compound (I) cr its salt can be prepared by reacting a 2-(4-hydroxyphenoxy)imidazo[1,2-

a]pyridine derivative (IV) or its salt with an alkanoic acid derivative (V) having a removable group W in the presence of a base. In this reaction, thc optically active compound (I) or its salt can be obtained by use of an optically active alkanoic acid derivative (V) or its salt.

The bases to be used in the reaction may be, for example, inorganic bases (e.g., alkali metal hydroxide or carbonate such as sodium hydroxide, potassium hydroxide, sodium carbonate, or alkali metal hydride such as sodium hydride). The reaction is preferably conducted in an inert solvent, for example, aromatic hydrocarbons such as benzene, toluene, etc., halogenated hydrocarbons such as dichloromethane, chloroform, carbon tetrachloride, etc., ethers such as diethyl ether, diisopropyl ether, dioxane, THF, etc., nitriles such as acetonitrile, etc., ketone such as acetone, methyl ethyl ketone, etc., esters such as ethyl acetate, butyl acetate, etc., dimethylformamide, dimethylsulfoxide, or the like.

The reaction temperature is usually 20 to 200 °C, preferably 80 to 150 °C. The reaction is completed in about 30 minutes to 20 hours, and the completion can be confirmed by thin layer chromatography or high pressure liquid chromatography.

The compounds (V) as the raw mateirals can be prepared by the method described in Journal of Pesticide Science 10, 69 (1985) or analogous methods thereto.

Route C:

The reaction can be summarized by the following scheme.

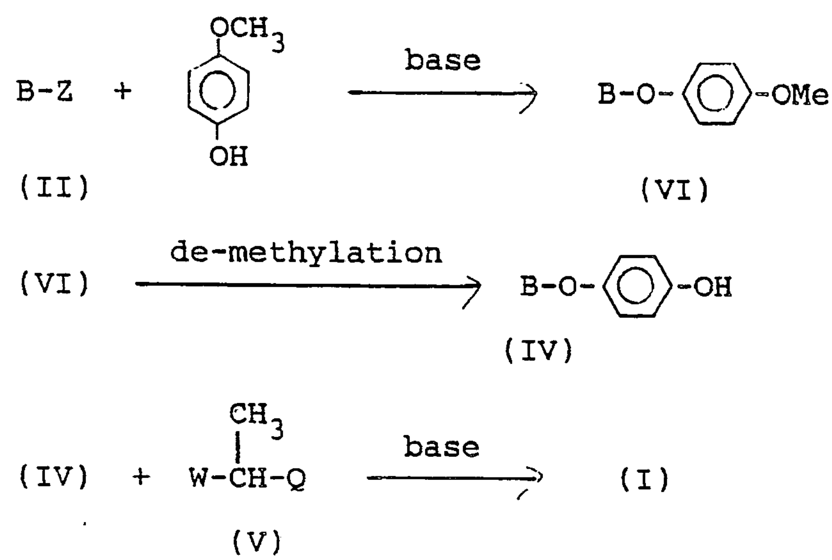

(II) (VI) (VI) (IV) (IV) (V) (I)

According to the Route C, 2-(4-methoxyphenoxy)imidazo[1,2-a]pyridine derivatives (VI) or their salts can be prepared by reacting an imidazo[1,2-a]pyridine compound (II) having a removable group at the 2nd position or its salt with p-methoxyphenol in the presence of a base. The bases and solvents to be used in the reaction may be the same as those of Route A and Route B, and accordingly any more explanation is not necessary. The reaction temperature and the reaction time are also similar to those of Routes A and B.

Thus obtained 2-(4-methoxyphenoxy)imidazo[1,2-a]pyridine derivative (VI) or its salt is demethylated by heating with pyridine•hydrochloride in acetic acid or with hydrogen bromide, or in accordance with the method described in Tetrahedron Letters, Page 1327 (1970) or analogous methods thereto, to give the corresponding 2-(4-hydroxyphenoxy)imidazo[1,2-a]pyridine derivative (IV).

The third step of the Route C [(IV) + (V) → (I)] is the same as the second step of the Route B, and accordingly any more explanation is not necessary.

Route D:

The reaction can be summarized in the following scheme.

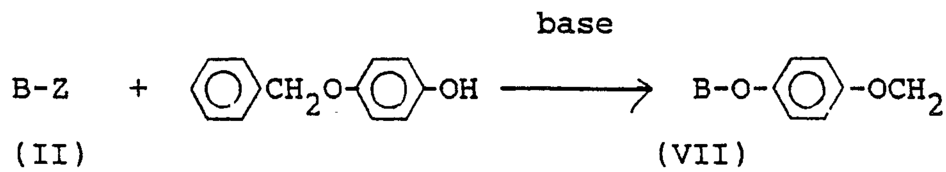


(II) (VII)

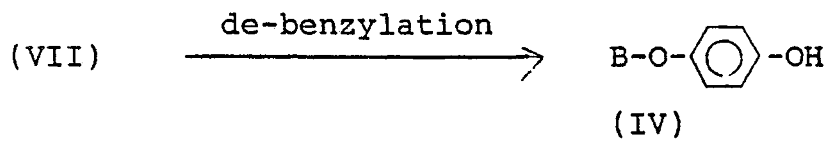


(VII) (IV)

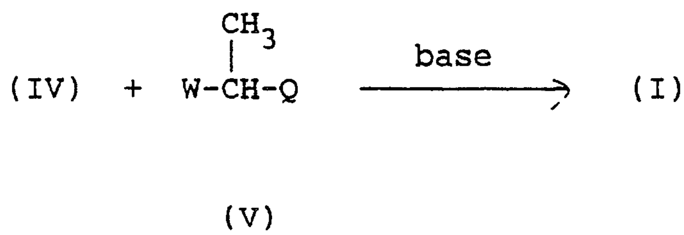


(IV) (I)

(V)

According to the Route D, 2-(4-benzyloxyphenoxy)imidazo[1,2-a]pyridine derivatives (VII) or their salts can be prepared by reacting an imidazo[1,2-a]pyridine compound (II) having a removable group at the 2nd position or its salt with p-benzyloxyphenol in the presence of a base.

The bases and solvents to be used in the reaction are the same as those of the Route A and Route B, and accordingly any additional explanation is not necessary. The reaction temperature and the reaction time are also the same as those of Routes A and B.

Thus obtained 2-(4-benzyloxyphenoxy)imidazo[1,2-a]pyridine derivative (VII) or its salt is subjected to a de-benzylation, for example, to treat with a metal catalyst (e.g., palladium on carbon, etc.) in a proper solvent (e.g., alcohols such as methanol, ethanol or n-propanol, ethers such as THF, dioxane or dimethoxyethane, or an optional mixture of these solvents), or with metallic sodium in t-butanol, thereby affording a 2-(4-hydroxyphenoxy)imidazo[1,2-a]pyridine derivative (IV) or its salt.

The third step of the Route D is the same as the second step of the Route B, and accordingly further explanation is not necessary.

Route E:

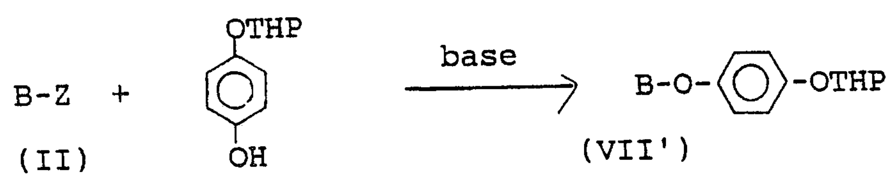


(II) (VII')

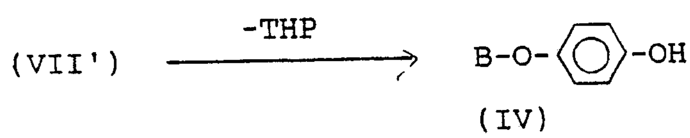


(VII') (IV)

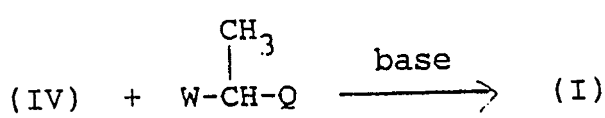


(IV) (I)

In the formulae, THP denotes tetrahydropyran-2-yl (hereinafter abbreviated as THP).

According to the first step of the Route E, 2-[4-(tetrahydropyran-2-yloxy)phenoxy]imidazo[1,2-a]pyridine derivatives (VII') or their salts can be prepared by treating an imidazo[1,2-a] pyridine compound (II) having a removable group at the 2nd position or its salt in the presence of a base. The bases and solvents to be

used and also the reaction temperature and reaction time are the same as those of the Route A and Route B, and accordingly any further explanation is not necessary.

Thus obtained 2-[4-(tetrahydropuran-2-yloxy)phenoxy]imidazo[1,2-a]pyridine derivative (VII′) or its salt is stirred in the presence of an acid catalyst such as p-toluenesulfonic acid, conc. sulfuric acid or conc. hydrochloric acid to give the corresponding 2-(4-hydroxyphenoxy)imidazo[1,2-a]pyridine derivative (IV) or its salt.

The third step of the Route E is the same as the second step of the Route B and accordingly any further explanation will be not required.

The compound (I) (wherein Q is not a cyano group) or its salt can also be prepared from a compound of the formula (I′):

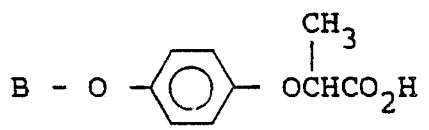

(I')

wherein B is as defined above.

For example, the compound (I) or its salt can be prepared by reacting a compound (I′) or its salt with a compound of the formula (VIII):

R - AH (VIII)

wherein R and A are the same as above in the presence of a proper dehydrating agent, alternatively by converting a compound (I′) or its salt to the corresponding acid halide (e.g., acid fluoride, acid chloride, acid bromide or acid iodide), and then treating the resulting acid halide with the compound (VIII) in the presence of a proper base.

The suitable dehydrating agents may be, for example, dicyclohexylcarbodiimide (hereinafter abbreviated as DCC), N,N′-carbonyldiimidazole, 2-halopyridinium salts such as 1-methyl-2-chlorpyridinium iodide, or the like.

This reaction using the dehydrating agent can be carried out in accordance with the method described in Newer Methods of Preparative Organic Chemistry (Academic Press, New York) Vol. 5, pages 61 - 108, J. Am. Chem. Soc. Vol. 80, page 6204 (1958), Chem. Lett. page 1045 (1975), etc., or analogous methods thereto.

The reaction for converting the compound (I′) or its salt to the acid halide can be conducted by the method described in, for example, "Synthesis and Reaction of Organic Compound (II)" pages 1104 -1120 in Shin Jikken Kagaku Koza, Vol. 14, edited by Japan Chemical Society, or analogous methods thereto.

The resulting acid halide is reacted with the compound (VIII) in the presence of a base such as organic tertiary amines (e.g., triethylamine or pyridine), or inorganic bases (e.g., sodium hydroxide or sodium carbonate) to give the compound (I) or its salt.

The reaction is conducted in an inert solvent: the suitable solvents may be, aromatic hydrocarbons such as benzene, toluene, etc., halogenated hydrocarbons such as dichloromethane, chloroform, carbon tetrachloride, etc., ethers such as diethyl ether, diisopropyl ether, dioxane, THF, etc., nitriles such as acetonitrile, etc., ketone such as acetone, methyl ethyl ketone, etc., esters such as ethyl acetate, butyl acetate, etc., dimethylformamide, dimethyl sulfoxide, or the like.

The reaction temperature is usually -10 to 100 °C, preferably 20 to 80 °C. The reaction is completed in about 30 minutes to 20 hours, and the completion can be confirmed by thin layer chromatography or high pressure liquid chromatography.

The compound (I) or its salt can also be prepared by converting a substituent shown by Y of the compound (I) or its salt to the other kind of substituent.

(1)

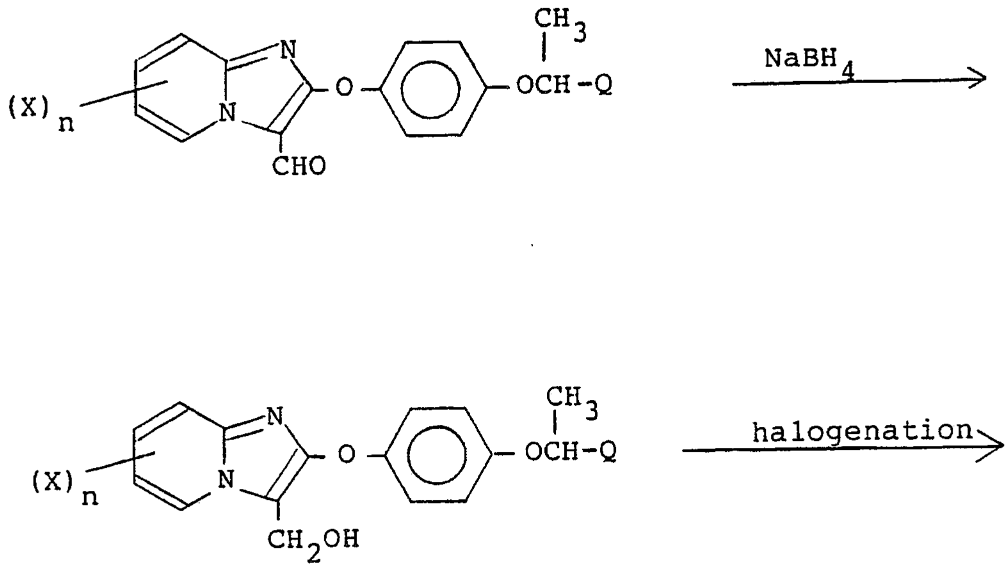

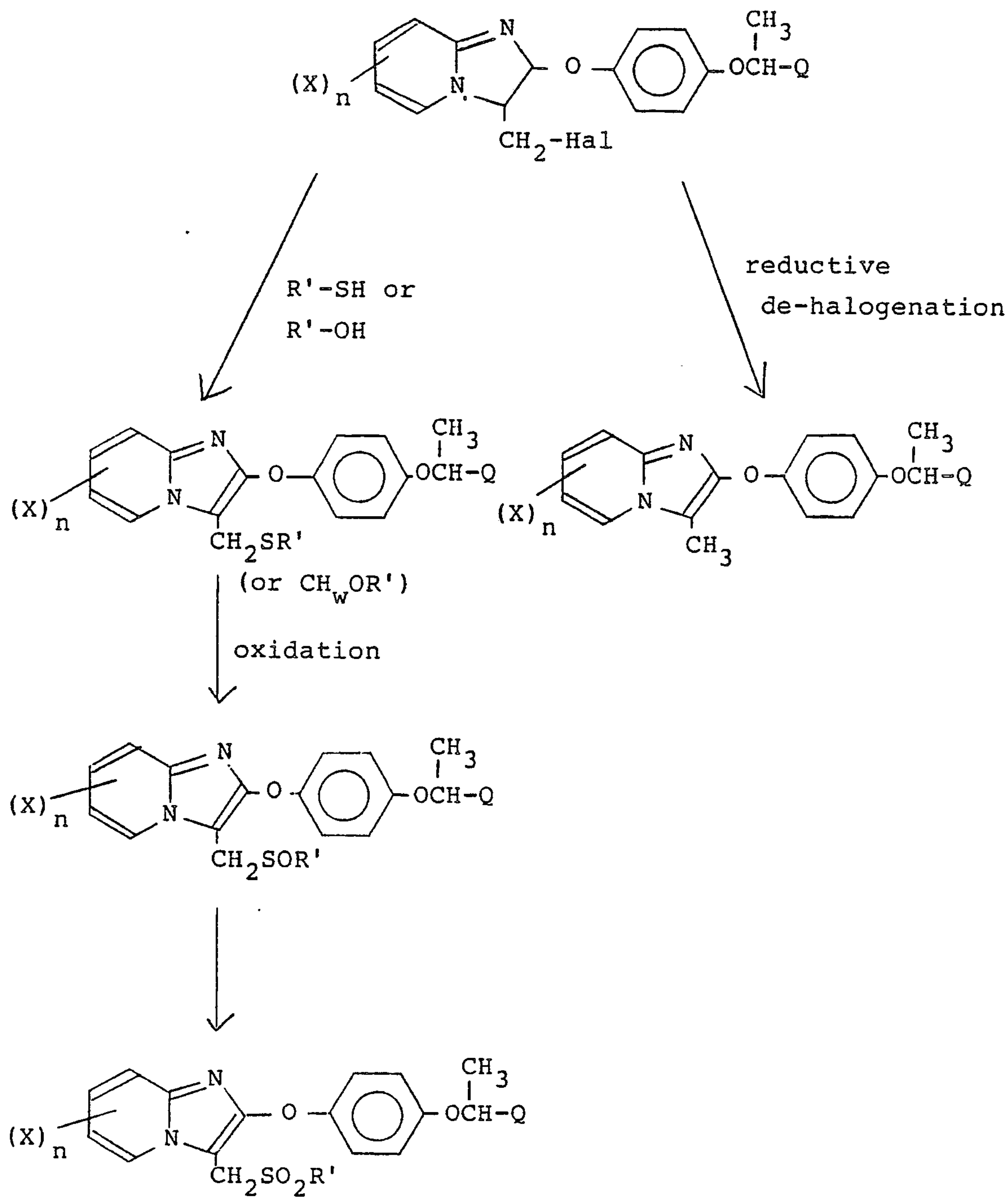


In the above formulae, X, Q and n have the same meanings as defined above and $R^1$ is a straight or branched chain $C_{1-6}$ alkyl.

(2)

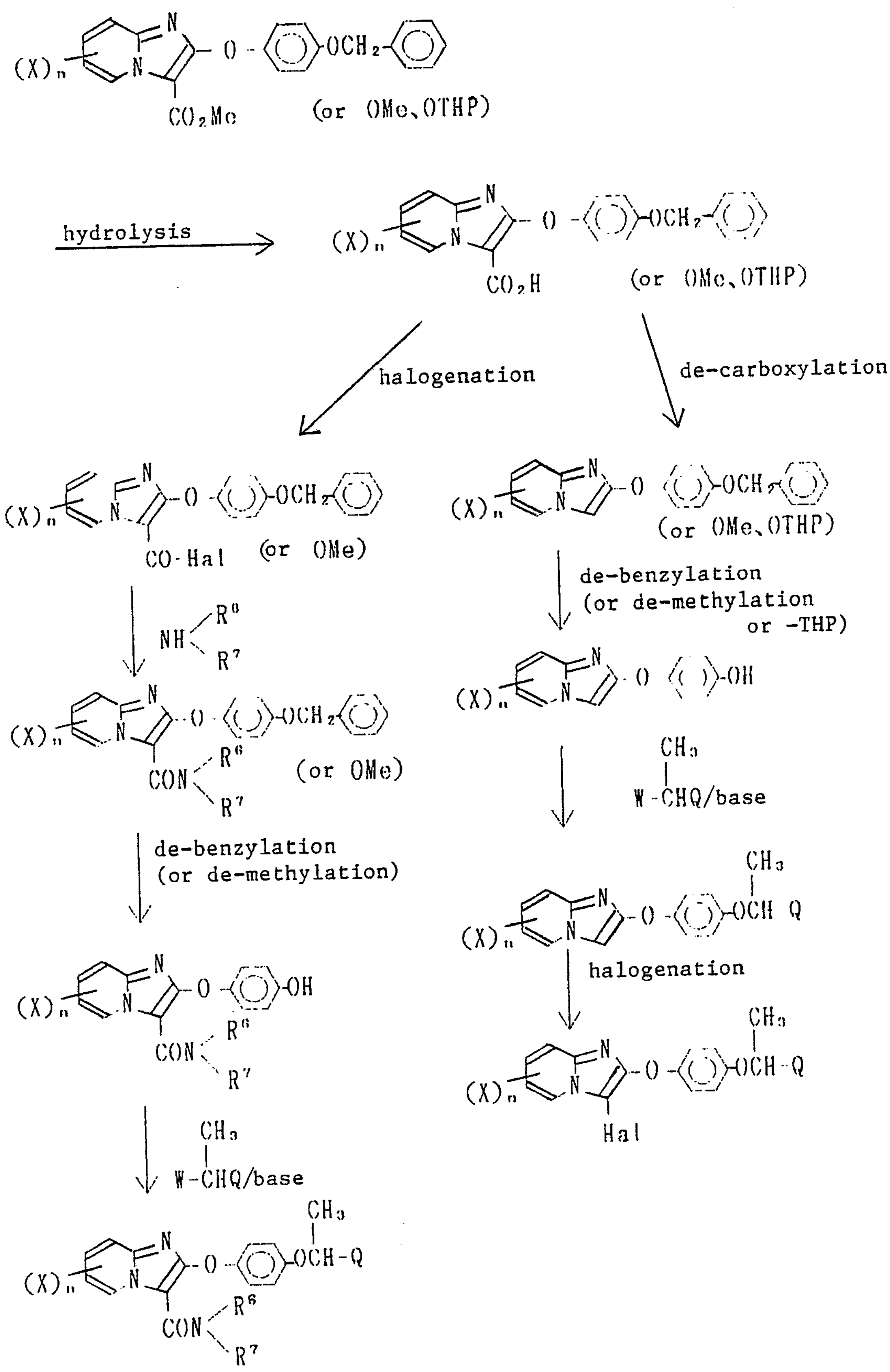
hydrolysis
halogenation
de-carboxylation
de-benzylation
(or de-methylation
or -THP)
/base
de-benzylation
(or de-methylation)
/base
halogenation

In the above formulae, $R^6$ and $R^7$ are hydrogen atom or a straight or branched chain $C_{1-6}$ alkyl group, and Me is methyl group. Also, "or OMe, OTHP" in the parenthesis below the formulae denotes to use OMe or OTHP instead of

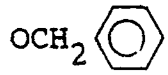 .

Thus obtained compound (I) or its salt can be isolated and purified by known methods such as concentration, change of basicity, distillation, extraction with solvent, salting out, crystallization, recrystallization and/or chromatography. Also, the free form or salt form of the compound (I) can be converted into the salt form or free form, respectively, in accordance with a conventional method.

The compounds (II), which are the starting materials for the compounds (I), can be prepared from a compound of the formula (II′) through the reactions shown in the following.

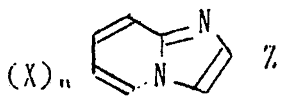

(II′)

(In the formula, X, Z and n have the same meanings as defined above.)

(1)

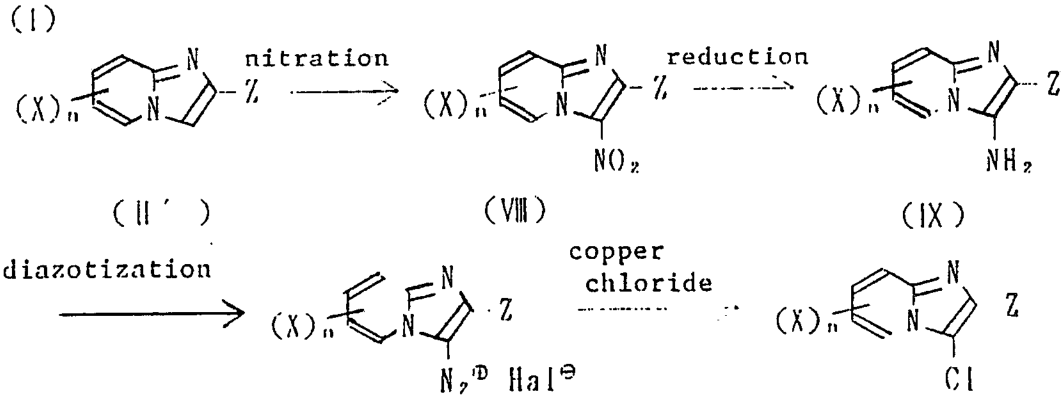


(II′) (VIII) (IX) (X) (XI)

(2)

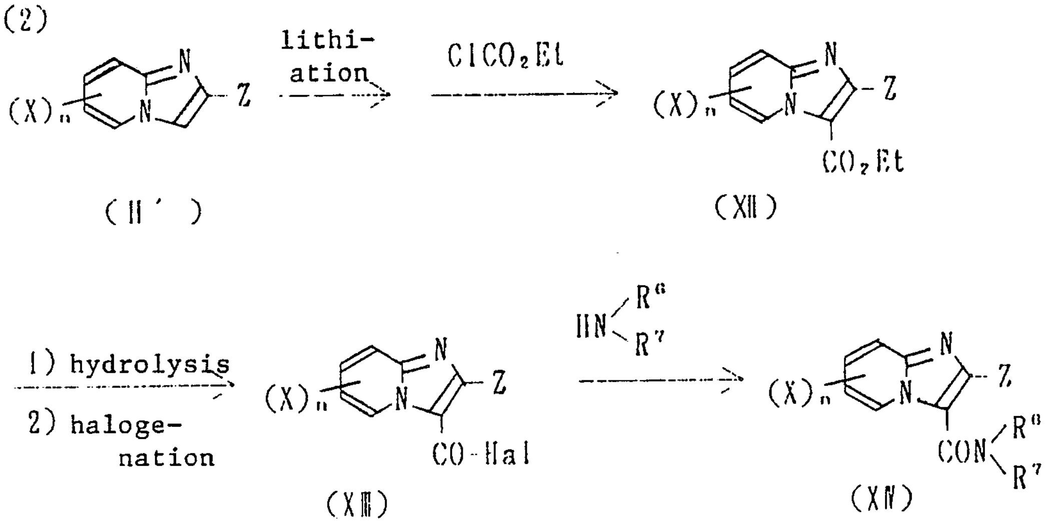


(II′) (XII) (XIII) (XIV)

(3)

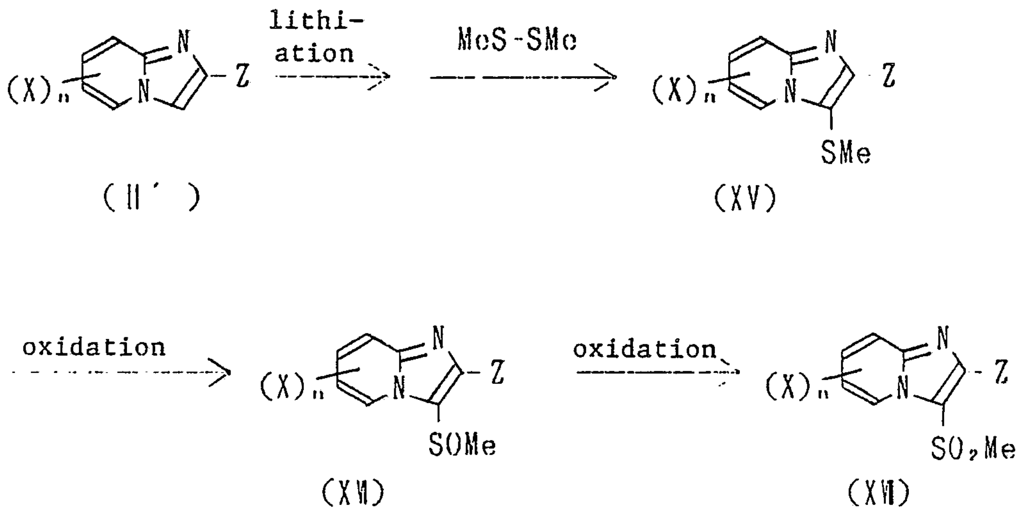


(II′) (XV) (XVI) (XVII)

(4)

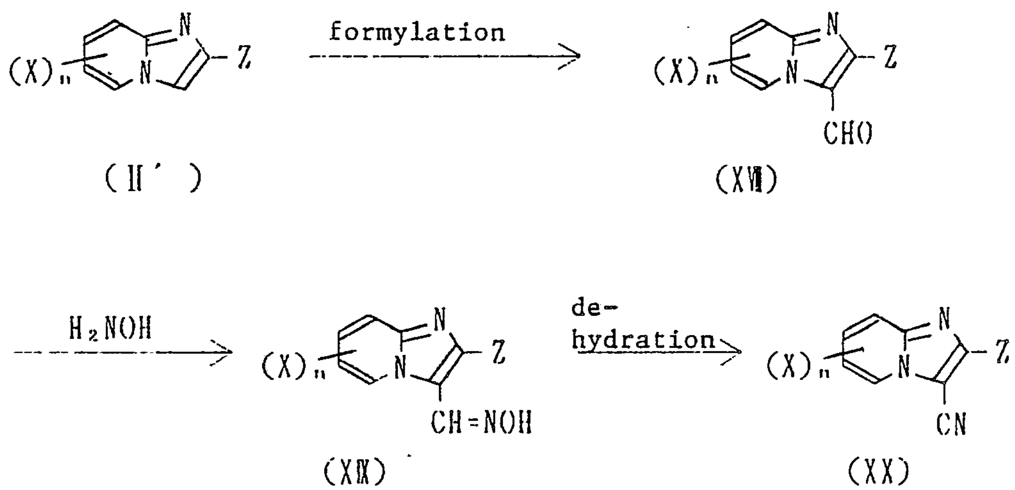


(II′) (XVIII) (XIX) (XX)

(5)

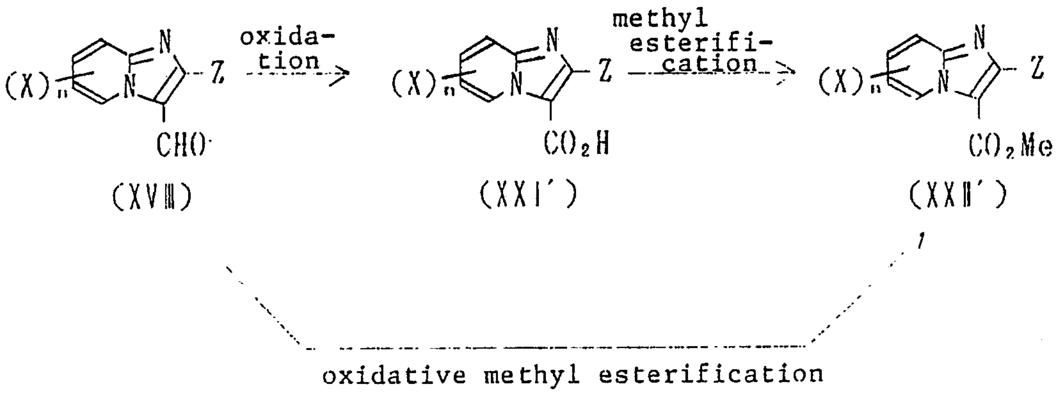


(XVIII) (XXI′) (XXII′)

(6)

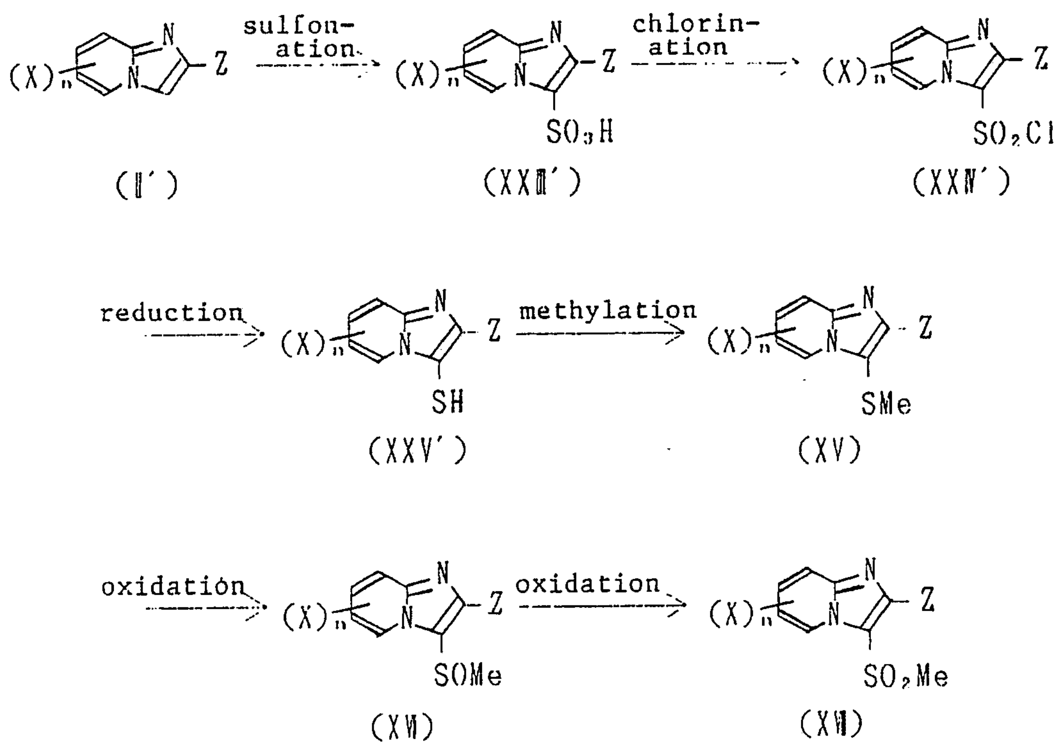


(II′) (XXIII′) (XXIV′) (XXV′) (XV) (XVI) (XVII)

The reactions included in the above (1) are known reactions. The nitration is achieved by use of a nitrating agent such as fuming nitric acid, conc. nitric acid, etc. This reaction is carried out in a solvent such as conc. sulfuric acid, acetic anhydride, etc., and the reaction temperature is usually about -20 to 100 °C. The reduction is conducted under a conventional condition for reducing nitro group, for example, by a method of catalytic hydrogenation. The compound (IX) is subjected to a conventional diazotization, for example, reacted with sodium nitrite in hydrochloric acid to give a diazonium salt (X), which is then reacted

with cuprous chloride to give a compound (XI).

In the above (2) and (3), hydrogen on the imidazole ring is substituted with lithium, and the resultant compound is reacted with an electrophilic reagent such as ethyl chlorocarbonate, dimethyldisulfide, etc., to give a compound (XII) or (XV), and then it is converted to the other kind of substituent. The lithiating agent to be used in these Routes may be alkyllithium such as methyllithium, n- butyllithium, t-butyllithium, etc., lithium amide, lithium diisopropylamide, or the like. The compound (XII) is hydrolyzed by a conventional method and then halogenated by a halogenating agent such as thionyl chloride, phosphorus oxychloride, etc., to give a compound (XIII).

The compound (XIII) is reacted with a primary or secondary amine in the presence of a base (e.g., pyridine, triethylamine, etc.) to give a compound (XIV). The compound (XV) is oxidized with a proper oxidizing agent such as hydrogen peroxide, potassium permanganate, m-chloroperbenzoic acid, etc., to give a compound (XVI), and then further oxidized to give a compound (XVII).

The reactions included in the above (4) are known reactions. The formylation can be conducted by a method described in Bulletin de la Societe Chemique de France, pages 1989 - 1999 (1962), J. Am. Chem. Soc. Vol. 75, page 989 (1953) or the like, or by a method similar thereto.

The compound (XVIII) is reacted with hydroxylamine to give a compound (XIX). This reaction is preferably carried out in an inert solvent such as dimethylformamide, acetonitrile, etc.

The compound (XIX) is reacted with a proper dehydrating agent such as phosphorus oxychloride, thionyl chloride, acetic anhydride, etc., to give a compound (XX).

The reactions included in the above (5) are known reactions. In the oxidation, an oxidating agent such as potassium permanganate, manganese dioxide, silver oxide, or the like can be used. The reaction is conducted in a solvent such as water or ethyl alcohol, usually at a temperature of 0 to 100 °C.

The methylesterification can be conducted under a conventional esterification condition, for example, is conducted in use of an acid catalyst such as conc. sulfuric acid, hydrochloric acid, p-toluenesulfonic acid, or the like in methanol.

The oxidative methylesterification of the compound (XVIII) can be conducted in accordance with the method described in Journal of Organic Chemistry, 54, 1213 (1987) or analogous methods thereto.

The reactions included in the above (6) are known reactions. In the sulfonation, a sulfonating agent such as sulfuric acid, fuming sulfuric acid, chlorosulfuric acid, etc. can be used. Also, a chlorinating agent such as thionyl chloride, phosphorus oxychloride, etc. can be used in the chlorination. The reduction can be conducted in accordance with the method described in Organic Synthesis Collective Volume 1, 504; or Canadian Journal of Chemistry, 41, 1646(1963), or analogous methods thereto. In the methylation, methyl iodide, dimethyl sulfate or the like can be used as a methylating agent.

The compounds (II′) and their salts used as the starting materials in the above reactions (1), (2), (3), (4) and (6) can be prepared by the method described in The Chemistry of Heterocyclic Compounds (Interscience Publishers) Vol. 15, Part 1 and Part 2, ibid Vol. 30, Comprehensive Heterocyclic Chemistry (Pergamon Press) Vol. 4, J. Heterocycl. Chem. Vol. 2, page 53 (1965), etc., or by analogous methods thereto.

And further, the compounds (II′) or their salts can be prepared by the reactions shown in the following.

(1)

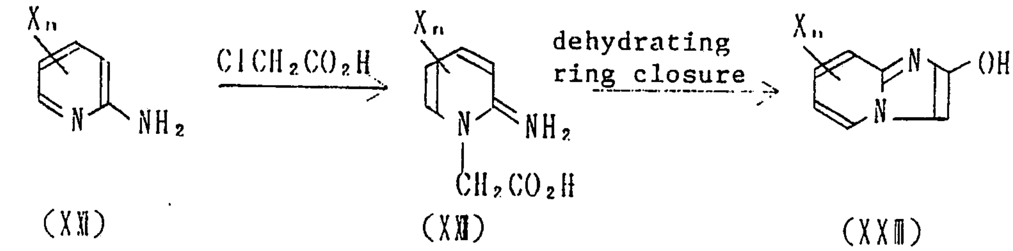


(XXI) (XXII) (XXIII)

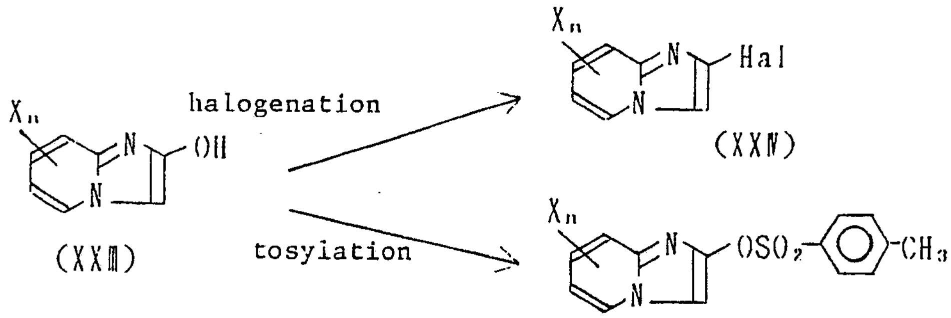


(XXIII) (XXIV) (XXV)

(wherein X and n are the same as above)

The reactions shown in the scheme (1) are carried out by the method described in Chem. Ber. Vol. 57, pages 1381 and 2091 (1924), or by methods similar thereto.

The compound (XXIII) is halogenated with a halogenating agent such as phosphorus chloride, thionyl chloride, etc., to give a compound (XXIV).

The compound (XXIII) is also reacted with p-toluenesulfonyl chloride in the presence of an appropriate base (e.g., pyridine, triethylamine, etc.) to give a compound (XXV).

According to the reaction scheme (1) and (2), the compound (XXIV) can also be prepared by reacting the compound (XXII) with a halogenating agent such as phosphorus oxychloride without going through the compound (XXIII).

The substituent X on the pyridine ring of the compound (II′) can be converted to the other kind of substituent by reactions shown in the following scheme.

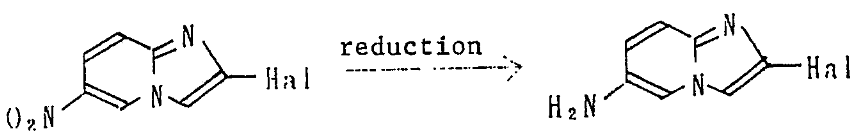


(XXVI) (XXVII)

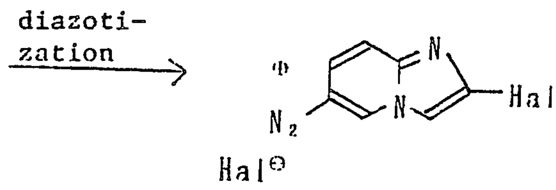


(XXVIII)

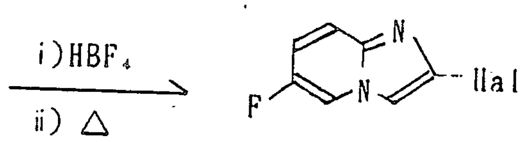


(XXIX)

The reactions included in the above are known. The compound (XXVI) is reduced by a conventional method, for example, catalytic hydrogenation to give the compound (XXVII). The steps from the compound (XXVII) to the compound (XXIX) can be conducted by the method described in Chem. Ber. Vol. 60, page 1189 (1972), or methods similar thereto. That is, the starting material for preparing the compounds of the present invention can be easily prepared by known methods or methods similar thereto. Also, the above-mentioned salts of the compounds (I) are applicable to the salts of the above starting materials or intermediate.

The compounds (I) of this invention and their salts including optical isomers thereof show excellent herbicidal activity in paddy field or the like and less damage against crops as shown below. Accordingly, excellent selective herbicides can be presented by this invention.

The present invention is further explained by the following Reference Examples, Examples and Formulation Examples, but this invention shall not be restricted by them.

The symbols in the Preparations and Examples possess the following meanings.

s : singlet
d : doublet
t : triplet
q : quartet
d,d : double doublet
m : multiplet
br : broad
J : coupling constant
DMSO : dimethylsulfoxide
Me : methyl group
Et : ethyl group
Ph : phenyl group

Reference Example 1

2-Chloro-3-ethoxycarbonylimidazo[1,2-a]pyridine

2-Chloroimidazo[1,2-a]pyridine (15.3g) was dissolved in anhydrous THF (100ml) under dried nitrogen gas stream, and the solution was cooled to -65° C. Then, n-butyllithium (1.6M hexane solution) (68.7ml) was added dropwise to the solution under -60° C for 40 minutes. The mixture was stirred for 30 minutes at -60° C or below, to which a solution of ethyl chlorocarbonate (11.9g) in anhydrous THF (10ml) was dropwise added, taking 25 minutes. The mixture was stirred for 1.5 hours at -60° C or below, and the reaction mixture was allowed to stand at the room temperature, poured into ice-water (about 0.5l) and then extracted with dichloromethane. The dichloromethane layer was separated and dried with anhydrous sodium sulfate. Dichloromethane was distilled off and the residue was purified by a silica gel column chromatography to give the captioned compound (16.4g). m.p. : 61 - 62° C
NMR($CDCl_3$)δ: 1.45(t,3H), 4.48(q,2H), 6.92-7.38 (m,1H), 7.50(dd,2H), 9.30(d,1H)

Reference Example 2

2,6-Dichloro-3-nitroimidazo[1,2-a]pyridine

2,6-Dichloroimidazo[1,2-a]pyridine (3.7g) was added to conc. sulfuric acid (18ml) under ice-cooling, and conc. nitric acid (2.5ml) was dropwise added to the mixture in 15 minutes with vigorous stirring. The mixture was stirred for 30 minutes under ice-cooling and for 3 hours at room temperature and then poured into ice-water (100ml). Precipitating crystals were collected by filtration, washed with water and dried to give the captioned compound (3.8g). m.p.: 171 - 172° C
NMR($CDCl_3$)δ: 7.70(s,2H), 9.51(s,1H)

Reference Example 3

2,6-Dichloro-3-formylimidazo[1,2-a]pyridine

Phosphorous oxychloride (6ml) was dropwise and slowly added to anhydrous dimethyl formamide (45ml) at 5 to 10° C under dried nitrogen gas atmosphere, followed by stirring for 15 minutes at a temperature below 5° C. And a solution of 2,6-dichloroimidazo[1,2-a]pyridine (10g) in dimethyl formamide (25ml) was dropwise added to the mixture at 5 to 10° C. The mixture was stirred at room temperature for 3 hours and poured into ice-water (about 0.5 liter). Precipitated crystals were collected by filtration, washed with water and dried to give the captioned compound (10.8g). m.p. 98 -98.5° C
NMR($CDCl_3$)δ: 7.60(s,2H), 8.54(s,1H), 9.96(s,1H).

Aldehyde compounds obtained by the method of Reference Example 3 are shown in the following Table 1.

Table 1

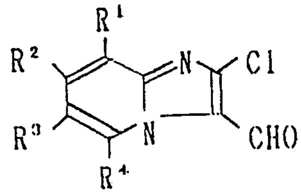

| $R^1$ | $R^2$ | $R^3$ | $R^4$ | NMR($CDCl_3$)(δ) |
|---|---|---|---|---|
| H | H | H | H | 6.90-7.50(m,1H), 7.52-7.90(m,2H), 9.44(d,1H), 10.0(s,1H) |
| H | H | Br | H | 7.50-7.80(m,2H), 9.68(s,1H), 10.01(s,1H) |
| H | H | $CF_3$ | H | 7.4-7.9(m,2H), 9.8(s,1H), 10.0(s,1H) |
| H | H | Me | H | 2.42(s,3H), 7.43(dd,1H), 7.62(d,1H), 9.32(s,1H), 9.95(s,1H) |
| Me | H | H | H | |
| H | Me | H | H | 2.51(s,3H), 7.02(d,1H), 7.48(s,1H), 9.38(d,1H), 9.98(s,1H) |
| H | H | H | Me | |

Reference Example 4

2,6,7-Trichloro-3-formylimidazo[1,2-a]pyridine

Phosphorus oxychloride (5.3g) was dropwise added to anhydrous dimethylformamide (50ml) under dried nitrogen gas atmosphere, and the mixture was stirred at 5 to 10°C for 20 minutes. 2,6,7-Trichloroimidazo[1,2-a]pyridine in crystalline form (5.16g) was added thereto, and the mixture was stirred at 35 to 40°C for 7 hours. The reaction mixture was allowed to stand overnight and poured into ice-water (about 0.3 liter). Precipitated crystals were collected by filtration, washed with water and dried to give the captioned compound (4.7g). m.p. 142 - 143.5°C

NMR($CDCl_3$)δ: 7.70(d,1H), 9.58(d,1H), 10.05(s,1H)

Reference Example 5

2,6-Dichloro-3-cyanoimidazo[1,2-a]pyridine

2,6-Dichloro-3-formylimidazo[1,2-a]pyridine (10g) and hydroxylamine hydrochloride (3.9g) were added to dimethylformamide (60ml). The mixture was stirred at 75 to 80°C for 3.5 hours and cooled to a temperature below 10°C, and then phosphorus oxychloride (8.8 ml) was dropwise added thereto. The mixture was stirred overnight at room temperature and poured into ice-water (about 0.5 liter). Precipitated crystals were collected by filtration, washed with water, dried and then recrystallized from ethyl acetate to obtain colorless needles of the captioned compound (7.8g). m.p. 155 - 159°C

NMR($CDCl_3$)δ: 7.50-7.70(m,2H), 8.30-8.48(m,1H)

Nitrile compounds obtained by the method similar to Reference Example 5 are shown in the following Table 2.

Table 2

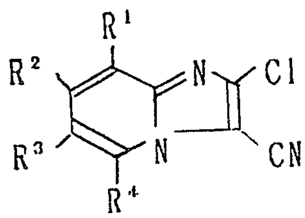

| $R_1$ | $R_2$ | $R_3$ | $R_4$ | NMR($CDCl_3$)(δ) |
|---|---|---|---|---|
| H | H | H | H | |
| H | H | Br | H | 7.62(s,2H), 8.45(s,1H) |
| H | H | $CF_3$ | H | 7.59(dd,1H), 7.88(d,1H), 8.68(s,1H) |
| H | H | Me | H | 2.45(s,3H), 7.39(d,1H), 7.60(d,1H), 8.09(s,1H) |
| Me | H | H | H | |
| H | Me | H | H | 2.50(s,3H), 7.0(d,1H), 7.45(s,1H), 8.15(d,1H) |
| H | H | H | Me | |
| H | Cl | Cl | H | 7.60(d,1H), 8.30(d,1H) |

Reference Example 6

2-(4-Benzyloxyphenoxy)-3-ethoxycarbonylimidazo[1,2-a]pyridine

60% Oily sodium hydride (0.7g) was suspended in anhydrous dimethylformamide (50ml). To the suspension was added slowly crystalline hydroquinone monobenzylether (3.5g), and the mixture was stirred

at room temperature for 30 minutes and then at 100°C for 10 minutes. Crystalline 2-chloro-3-ethoxycarbonylimidazo[1,2-a]pyridine (3.7g) was added to the mixture and stirred at 105 - 110°C for 8 hours. The reaction mixture was poured into ice-water (about 0.3l) and extracted with chloroform. The chloroform layer was separated and dried with anhydrous sodium sulfate. Chloroform was distilled off and the residue was purified by a silica gel column chromatography to give the captioned compound (2.8g). m.p. 119 - 120°C

Reference Example 7

2-(4-Benzyloxyphenoxy)imidazo[1,2-a]pyridine

2-(4-Benzyloxyphenoxy)-3-ethoxycarbonylimidazo[1,2-a]pyridine (1.5g) was dissolved in ethanol (15ml). To the solution was added 10% aqueous solution of sodium hydroxide (10ml), and the mixture was stirred at room temperature for 40 minutes and refluxed under heating for 10 minutes with stirring. The reaction mixture was concentrated to remove ethanol. Water (about 50ml) was added to the residue and neutralized with conc. hydrochloric acid. Precipitated crystals were collected by filtration and dried to give colorless crystals. The crystals were heated in a round-bottom flask at 140 to 150°C for 20 minutes. After vigorous foaming, oily substance was produced, which was cooled to room temperature, dissolved in chloroform and washed with water. The chloroform layer was separated and dried with anhydrous sodium sulfate. The solvent was distilled off and the residue was purified by a silica gel column chromatography to give brown needles of the captioned compound (1.05g). m.p. 124.5 - 125.5°C.
NMR($CDCl_3$)δ: 5.05(s,2H), 6.75(t,2H), 6.90-7.09 (m,3H), 7.10-7.29(m,3H), 7.40(s,5H), 7.99(d,1H)

Reference Example 8

2-(4-Hydroxyphenoxy)imidazo[1,2-a]pyridine

2-(4-Benzyloxyphenoxy)imidazo[1,2-a]pyridine (1.9g) was dissolved in a mixed solvent of tetrahydro furan (25ml) and ethanol (25ml). To the mixture was added 10% palladium on carbon catalyst (0.2g) and hydrogenated for 12 hours. The reaction mixture was filtered to remove the catalyst and the filtrate was condensed. The resultant residue was purified by a silica gel column chromatography to give colorless crystals of the captioned compound (0.3g). m.p. 163 -164°C
NMR(D6-DMSO) δ: 6.70-7.51(m,7H), 8.41(d,1H), 9.30(s,1H)

Reference Example 9

2-(4-Hydroxyphenoxy)-3-ethoxycarbonylimidazo[1,2-a]pyridine

Colorless crystals of the captioned compound were obtained from 2-(4-benzyloxyphenoxy)-3-ethoxycarbonylimidazo[1,2-a]pyridine in a similar manner to Reference Example 8. m.p. 186 - 187°C

Reference Example 10

2,6-Dichloro-3-methoxycarbonylimidazo[1,2-a]pyridine

To a suspension of 2,6-dichloro-3-formylimidazo[1,2-a]pyridine (3.2g) in anhydrous methanol (100ml) were added N-iodosuccinimide (10.1g) and potassium carbonate (5.2g). The mixture was stirred for 24 hours at room temperature in dark place. Then, a solution of sodium thiosulfate (7.0g) in water (150ml) was added to the reaction mixture and vigorously stirred for about 20 minutes. The resulting solution was extracted with chloroform. The collected chloroform layer was dried over anhydrous sodium sulfate and

distilled to remove chloroform. The residue was dissolved in ethanol (60ml), and Girard's reagent (1.3g) and conc. hydrochloric acid (a few drops) were added to the solution and refluxed for 15 minutes. The precipitated crystals were filtered off and the filtrate was concentrated under reduced pressure to obtain colorless crystals of the title compound (1.5g). m.p. 133 - 134 °C
NMR($CDCl_3$)δ: 4.0(s,3H), 7.41(dd,1H), 7.59(d,1H), 9.42(d,1H)

Reference Example 11

2-(4-methoxyphenoxy)-6-chloro-3-methoxycarbonylimidazo[1,2-a]pyridine

To a suspension of 60% sodium hydride in oil (0.84g) in anhydrous dimethylformamide (60ml) was added p-methoxyphenol in crystalline form (2.6g). The mixture was stirred for 20 minutes at room temperature and for 10 minutes at about 60 °C. Then 2,6-dichloro-3-methoxycarbonylimidazo[1,2-a]pyridine in crystalline form (4.9g) was added to the mixture and stirred for 5 hours at 135 to 140 °C. The reaction mixture was poured into ice-water (about 300ml), acidified with conc. hydrochloric acid and extracted with dichloromethane. The residue was purified by a silica gel column chromatography to obtain colorless crystals of the title compound (1.9g). m.p. 162 - 165 °C.
NMR($CDCl_3$)δ: 3.80(s,3H), 3.98(s,3H), 6.91(d,2H), 7.21(d,2H), 7.32-7.49(m,2H), 9.43(s,1H)

Reference Example 12

2-(4-methoxyphenoxy)-6-chloroimidazo[1,2-a]pyridine

To a solution of 2-(4-methoxyphenoxy)-6-chloro-3-methoxycarbonylimidazo[1,2-a]pyridine (2.3g) in ethanol (35ml) was added 5% sodium hydroxide aqueous solution (20ml). The mixture was refluxed for an hour and distilled to remove the solvent. The residue to which water (50ml) was added was neutralized with conc. hydrochloric acid. The resulting crystals were collected by filtration and washed with water. The crystals was heated at 180 to 190 °C in round-bottom flask (100 liter) until generation of carbon dioxide gas is stopped. The residue was dissolved in chloroform (about 60ml) and the solution was filtered to remove insoluble material. The filtrate was concentrated, and the residue was purified by a silica gel column chromatography to obtain colorless crystals of the title compound (1.15g). m.p. 122 - 124 °C
NMR($CDCl_3$)δ: 3.80(s,3H), 6.89(d,2H), 6.98(s,1H), 7.00-7.30(m,3H), 7.41(d,1H), 8.05(s,1H)

Reference Example 13

2-(4-Hydroxyphenoxy)-6-chloroimidazo[1,2-a]pyridine

60% sodium hydride in oil (0.36g) was suspended in anhydrous dimethylformamide (10ml) under dry nitrogen gas stream. A solution of ethylmercaptan (0.57g) in dimethylformamide (10ml) was dropwise added to the suspension and stirred for 5 minutes at room temperature. Then, a solution of 2-(4-methoxyphenoxy)-6-chloroimidazo[1,2-a]pyridine (1.0g) in dimethylformamide (10ml) was added to the above mixture, and refluxed for 3 hours with stirring. The reaction mixture was cooled to room temperature and filtered. The filtrate was concentrated, and the residue was added with water (50ml), neutralized with conc. hydrochloric acid and extracted with ethyl acetate. The extract was dried over anhydrous magnesium sulfate and distilled to remove ethyl acetate. The residue was purified by a silica gel column chromatography to obtain pale yellowish crystals of the title compound (0.5g). m.p. 140 - 143 °C
NMR($d_6$-DMSO)δ: 6.80(d,2H), 6.96-7.23(m,4H), 7.38(d,1H), 8.29(s,1H), 9.01(s,1H)

Reference Example 14

2-[4-(Tetrahydropyran-2-yloxy)phenoxy]-6-chloro-3-cyanoimidazo[1,2-a]pyridine

To a suspension of 60% sodium hydride in oil (0.66g) in anhydrous dimethylformamide (10ml) was dropwise added a solution of p-(tetrahydropyran-2-yloxy)phenol (2.9g) in dimethylformamide (10ml) at room temperature. The mixture was stirred for 40 minutes at room temperature, to which 2,6-dichloro-3-cyanoimidazo[1,2-a]pyridine in crystalline form (2.1g) was added and stirred for 1.5 hours at 130 to 135° C. The reaction mixture was concentrated under reduced pressure, and the residue was treated with chloroform to remove insoluble material. The chloroform layer was distilled to remove chloroform. The residue was purified by a silica gel column chromatography to obtain colorless crystals of the title compound (1.9g). m.p. 172 - 175° C

NMR($CDCl_3$)δ: 1.33-2.12(br,m,6H), 3.38-3.71(br,m,1H), 3.75-4.10(br.m,1H), 5.31-5.44(m,1H), 6.93-7.31-(m,4H), 7.35-7.60(m,2H), 8.25(s,1H)

Reference Example 15

2-(4-Hydroxyphenoxy)-6-chloro-3-cyanoimidazo[1,2-a]pyridine

To a suspension of 2-[4-(tetrahydropyran-2-yloxy)phenoxy]-6-chloro-3-cyanoimidazo[1,2-a]pyridine (1.7g) in methanol (35ml) was added p-toluenesulfonic acid (0.09g). The mixture was stirred for 2 hours at room temperature and then filtered to collect crystals. The crystals were washed with chloroform to obtain colorless crystals of the title compound (1.1g). m.p. 280 - 283° C (decomp.)

NMR(DMSO-$d_6$)δ: 6.82(d,2H), 7.13(d,2H), 7.62(s,2H), 8.73(s,1H), 9.42(br.s.1H)

Example 1

Ethyl 2-[4-(3-nitroimidazo[1,2-a]pyridin-2-yloxy)phenoxy]propionate (Compound No. 1)

Ethyl 2-(4-hydroxyphenoxy)propionate (4.3g) was dissolved in acetonitrile (60ml). To the solution was added potassium carbonate (2.8g) and the mixture was refluxed under heating for 30 minutes. 2-Chloro-3-nitroimidazo[1,2-a]pyridine (4.0g) was added to the mixture and refluxed under heating for 4.5 hours. The reaction mixture was filtered and the filtrate was concentrated. The residue was dissolved in dichloromethane and washed with water. The dichloromethane layer was separated and dried with anhydrous sodium sulfate. Dichloromethane was distilled off, and the residue was recrystallized from ethanol to give yellow crystals of the captioned compound (7.0g). m.p. 125 -127° C.

NMR($CDCl_3$)δ: 1.25(t,3H), 1.60(d,3H), 4.23(q,2H), 4.75(q,1H), 6.71-7.10(m,2H), 7.10-7.42(m,3H), 7.50-7.90-(m,2H), 9.49(d,1H)

Example 2

Ethyl 2-[4-(3-nitro-6-chloroimidazo[1,2-a]pyridin-2-yloxy)phenoxy]propionate (Compound No. 2)

2,6-Dichloro-3-nitroimidazo[1,2-a]pyridine was treated in a similar manner to Example 1 to give colorless crystals of the captioned compound. m.p. 121 - 122.5° C

NMR($CDCl_3$)δ: 1.24(t,3H), 1.65(d,3H), 4.23(q,2H), 4.75(q,1H), 6.94(d,2H), 7.28(d,2H), 7.57(s,2H), 9.55(s,1H)

Example 3

Ethyl 2-[4-(imidazo[1,2-a]pyridine-2-yloxy)phenoxy]propionate (Compound No. 3)

2-(4-Hydroxyphenoxy)imidazo[1,2-a]pyridine (0.27g) was suspended in acetonitrile (10ml). Potassium

carbonate (0.17g) was added to the suspension, and the mixture was refluxed under heating for 1 hour. To the mixture was added ethyl α-bromopropionate (0.22g) and the mixture was further refluxed for 7 hours. The reaction mixture was filtered and the filtrate was concentrated. The resultant residue was purified by a silica gel column chromatography to give yellow oil of the captioned compound (0.37g).
NMR($CDCl_3$)δ: 1.18(t,3H), 1.60(d,3H), 4.21(q,2H), 4.70(q,1H), 6.60-7.65(m,8H), 8.0(d,1H)

Example 4

Ethyl 2-[4-(3-ethoxycarbonylimidazo[1,2-a]pyridin-2-yloxy)phenoxy]propionate (Compound No. 4)

2-(4-Hydroxyphenoxy)-3-ethoxycarbonylimidazo[1,2-a]pyridine was treated in a similar manner to the Example 3 to give colorless crystals of the captioned compound. m.p. 98 - 99 °C
NMR($CDCl_3$)δ: 1.22(t,3H), 1.35(t,3H), 1.60(d,3H), 4.21(q,2H), 4.41(q,2H), 4.70(q,1H), 6.80-7.59(m,7H), 9.35-(d,1H)

Example 5

Ethyl 2-[4-(3-cyanoimidazo[1,2-a]pyridin-2-yloxy)phenoxy]propionate (Compound No. 5)

Ethyl 2-(4-hydroxyphenoxy)propionate (1.2g) was dissolved in dimethyl sulfoxide (30ml). To the solution was added potassium carbonate (0.8g), and the mixture was stirred at 80 °C for 30 minutes. 2-Chloro-3-cyanoimidazo[1,2-a]pyridine (1.0g) was added to the mixture, and the mixture was heated to 140 to 150 °C and stirred for 4 hours. The reaction mixture was poured into ice-water (about 0.2l), weakly acidified with hydrochloric acid and extracted with diethyl ether. The ether layer was separated, washed with water several times and then dried over anhydrous sodium sulfate. The ether was distilled off, and the resultant redisue was purified by a silica gel column chromatography to give colorless crystals of the captioned compound (0.2g). m.p. 144 - 146 °C.
NMR($CDCl_3$)δ: 1.25(t,3H), 1.64(d,3H), 4.25(q,2H), 4.77(q,1H), 6.85-7.10(m,3H), 7.12-7.38(m,2H), 7.51(t,2H), 8.25(d,1H)

Example 6

2-[4-(3-Carbamoylimidazo[1,2-a]pyridin-2-yloxy)phenoxy]propionic acid (Compound No. 6)

Ethyl 2-[4-(3-cyanoimidazo[1,2-a]pyridin-2-yloxy)phenoxy]propionate (4.6g) was dissolved in ethanol (50ml). To the suspension was added 10% aqueous solution of sodium hydroxide (10ml), and the mixture was stirred at room temperature for 1.5 hours, and refluxed for 1 hour. The reaction mixture was filtered to give crystals, which were dissolved in water and neutralized with conc. hydrochloric acid. The precipitated crystals were collected by filtration to give the captioned compound (3.0g).
IR(nujol)$cm^{-1}$: 3500, 3250, 3167, 1700

Example 7

2-[4-(3-Carbamoylimidazo[1,2-a]pyridin-2-yloxy)phenoxy]propionanilide (Compound No. 7)

2-[4-(3-Carbamoylimidazo[1,2-a]pyridin-2-yloxy)phenoxy]propionic acid (1.3g) and 1-methyl-2-chloropyridinium iodide (1.03g) were suspended in dichloromethane (15ml). To the suspension were dropwise added in 10 minutes a solution of aniline (0.37g) in dichloromethane (5ml) and a solution of triethylamine (0.41g) in dichloromethane (5ml) at the same time at room temperature. After stirring at room temperature for 4.5 hours, chloroform (100ml) was added to the reaction mixture and washed with water 5

times. The chloroform layer was separated and dried with anhydrous sodium sulfate. Chloroform was distilled off, and the residue was purified by a silica gel column chromatography to give colorless crystals of the captioned compound (0.5g). m.p. 208 - 207° C
NMR($CDCl_3$)δ: 1.65(d,3H), 4.75(q,1H), 6.10(br,s,2H), 6.80-7.80(m,12H), 9.62(d,1H)

Example 8

Ethyl 2-[4-(3-cyano-6-chloroimidazo[1,2-a]pyridin-2-yloxy)phenoxy]propionate (Compound No. 8)

60% Oily sodium hydride (0.4g) was suspended in anhydrous dimethyl formamide (20ml). To the suspension was dropwise added a solution of ethyl 2-(4-hydroxyphenoxy)propionate (2.1g) in dimethyl formamide (10ml) at room temperature. After stirring for 20 minutes, crystalline 2,6-dichloro-3-cyanoimidazo[1,2-a]pyridine (2.1g) was added to the mixture, heated to 120° C and stirred for 4 hours. The reaction mixture was poured into ice-water (about 0.3l) and extracted with chloroform. The chloroform layer was washed with water 4 times, separated and dried with anhydrous sodium sulfate. Chloroform was distilled off, and the residue was purified by a silica gel column chromatography to give colorless crystals of the captioned compound (1.8g). m.p. 111 - 112° C
NMR($CDCl_3$)δ: 1.25(t,3H), 1.61(d,3H), 4.22(q,2H), 4.75(q,1H), 6.91(d,2H), 7.20(d,2H), 7.32-7.51 (m,2H), 8.29-(s,1H)

Example 9

2-[4-(3-Cyano-6-chloroimidazo[1,2-a]pyridin-2-yloxy)phenoxy]propionic acid (Compound No. 9)

Ethyl 2-[4-(3-cyano-6-chloroimidazo[1,2-a]pyridin-2-yloxy)phenoxy]propionate (2.4g) was suspended in ethanol (15ml). A solution of sodium hydroxide (0.36g) in water (5ml) was added to the suspension and stirred overnight at room temperature. The reaction mixture was neutralized with conc. hydrochloric acid, and the precipitating crystals were collected by filtration to give colorless crystals of the captioned compound (1.6g). m.p. 173 - 177° C (decomp.)
NMR(DMSO-$d_6$)δ: 1.53(d,3H), 4.82(q,1H), 6.95(d,2H), 7.29(d,2H), 7.68(s,2H), 8.80(s,1H), 12.85(br,s,1H)

Example 10

Methyl 2-[4-(3-cyano-6-chloroimidazo[1,2-a]pyridin-2-yloxy)phenoxy]propionate (Compound No. 10)

2-[4-(3-Cyano-6-chloroimidazo[1,2-a]pyridin-2-yloxy)phenoxy]propionic acid (0.6g) was suspended in dichloromethane (5ml). N,N′-Carbonyldiimidazole (0.4g) was added to the suspension and stirred at room temperature for 1 hour. Methanol (0.2ml) was added to the mixture and stirred at room temperature for 2 hours. Dichloromethane (30ml) was added to the reaction mixture, and washed with dil. hydrochloric acid and then with water. The dichloromethane layer was separated and dried with anhydrous sodium sulfate. Dichloromethane was distilled off, and the resultant residue was purified by a silica gel column chromatography to give colorless crystals of the captioned compound (0.42g). m.p. 151 - 152° C
NMR($CDCl_3$)δ: 1.62(d,3H), 3.79(s,3H), 4.78(q,1H), 6.95(d,2H), 7.25(d,2H), 7.40-7.61 (m,2H), 8.30(s,1H)

Example 11

N-Methyl-N-phenyl-2-[4-(3-cyano-6-chloroimidazo[1,2-a]pyridin-2-yloxy)phenoxy]propionamide (Compound No. 11)

2-[4-(3-Cyano-6-chloroimidazo[1,2-a]pyridin-2-yloxy)phenoxy]propionic acid (0.6g) was suspended in

dichloromethane (10ml). N,N′-Carbonyldiimidazole (0.5g) was added to the suspension and stirred at room temperature for 1 hour. N-Methylaniline (0.18g) was added to the mixture, stirred at room temperature for 2 hours, refluxed under heating for 3 hours, and then allowed to stand at room temperature for 2 weeks. Dichloromethane (50ml) was added to the reaction mixture and washed with water. The dichloromethane layer was separated and dried with anhydrous sodium sulfate. Dichloromethane was distilled off, and the residue was purified by a silica gel column chromatography to give colorless crystals of the captioned compound (0.4g). m.p. 174 - 175 °C
NMR($CDCl_3$)δ: 1.51(d,3H), 3.32(s,3H), 4.79(q,1H), 6.81(d,2H), 7.10-7.68(m,9H), 8.35(s,1H)

Example 12

Ethyl 2-[4-(6-chloroimidazo[1,2-a]pyridin-2-yloxy) phenoxy]propionate (Compound No. 20)

The title compound as pale yellowish oil was prepared from 2-(4-hydroxyphenoxy)-6-chloroimidazo[1,2-a]pyridine in accordance with the method of Example 3.
NMR($CDCl_3$)δ: 1.22(t,3H), 1.60(d,3H), 4.21(q,2H), 4.69(q,1H), 6.90(d,2H), 6.99(s,1H), 7.14(d,2H), 7.23(d,1H), 7.42(d,1H), 8.05(s,1H)

Example 13

Ethyl 2-[4-(3,6-dichloroimidazo[1,2-a]pyridin-2-yloxy)phenoxy]propionate (Compound No. 22)

To a solution of ethyl 2-[4-(6-chloroimidazo[1,2-a]pyridin-2-yloxy)phenoxy]propionate (0.6g) in chloroform (15ml) was added N-chlorosuccinimide (0.22g) at room temperature. After stirring for 2 hours, the reaction mixture was concentrated. The residue was purified by a silica gel column chromatography to obtain pale yellowish oil of the title compound (0.54g).
NMR($CDCl_3$)δ: 1.25(t,3H), 1.60(d,3H), 4.20(q,2H), 4.70(q,1H), 6.89(d,2H), 7.15(d,2H), 7.23(dd,1H), 7.41(d,1H), 8.05(d,1H)

Example 14

Ethyl R-(+)-2-[4-(3-cyano-6-chloroimidazo[1,2-a]pyridin-2-yloxy)phenoxy]propionate (Compound No. 221)

2-(4-Hydroxyphenoxy)-6-chloro-3-cyanoimidazo[1,2-a]pyridine (1.0g), potassium carbonate (0.48g) and ethyl O-methanesulfonyl-(S)-(-)-lactate (0.7g) were added to anhydrous dimethyl sulfoxide (10ml) and stirred for 48 hours at room temperature. The reaction mixture was filtered, and ethyl ether (200ml) was added to the filtrate. Then, the mixture was washed twice with water. The separated ether layer was dried over anhydrous magnesium sulfate and distilled to remove ether. The residue was purified by a silica gel column chromatography to obtain colorless crystals of the title compound (0.9g).
$[\alpha]_D^{22}$ = + 23.6° ($CHCl_3$, c = 2.0%)
IR (nujol) $cm^{-1}$: 2210, 1740
NMR($CDCl_3$)δ: 1.25(t,3H,J=6.3), 1.63(d,3H,J=6.3), 4.22(q,2H,J=6.3), 4.84(q,1H,J=6.3), 6.92(d,2H,J=9.0), 7.22(d,2H,J=9.0), 7.40-7.61(m,2H), 8.29(d,1H,J=1.5)

The optical purity of the product was ≧ 71% ee by $^1$HNMR analysis in the presence of Eu(hfc)$_3$.

The compounds listed in the following Table 3 are obtained in a similar manner to those of the above Examples 1 - 13.

Table 3

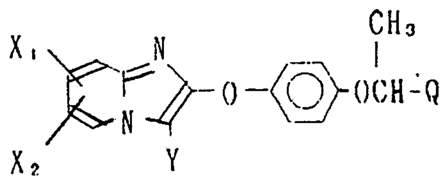

| Compound No. | $X_1$ | $X_2$ | Y | Q | m.p.(℃) |
|---|---|---|---|---|---|
| 12 | H | H | F | $CO_2Et$ | |
| 13 | H | H | Cl | $CO_2Et$ | |
| 14 | H | H | Br | $CO_2Et$ | |
| 15 | H | H | $COCH_3$ | $CO_2Et$ | |
| 16 | H | H | $CONMe_3$ | $CO_2Et$ | |
| 17 | H | H | CHO | $CO_2Et$ | |
| 18 | H | H | $CH_3$ | $CO_2Et$ | |
| 19 | H | H | $CF_3$ | $CO_2Et$ | |
| 20 | 6-Cl | H | H | $CO_2Et$ | |
| 21 | 6-Cl | H | F | $CO_2Et$ | |
| 22 | 6-Cl | H | Cl | $CO_2Et$ | |
| 23 | 6-Cl | H | Br | $CO_2Et$ | |
| 24 | 6-Cl | H | $COCH_3$ | $CO_2Et$ | |

Table 3 (continued)

| Compound No. | $X_1$ | $X_2$ | Y | Q | m.p. (°C) |
|---|---|---|---|---|---|
| 25 | 6-Cl | H | CHO | $CO_2Et$ | 92~93 |
| 26 | 6-Cl | H | SMe | $CO_2Et$ | |
| 27 | 6-Cl | H | SOMe | $CO_2Et$ | |
| 28 | 6-Cl | H | $SO_2Me$ | $CO_2Et$ | |
| 29 | 6-Cl | H | $CH_3$ | $CO_2Et$ | |
| 30 | 6-Cl | H | $CF_3$ | $CO_2Et$ | |
| 31 | 6-Cl | H | $SO_2NMe_2$ | $CO_2Et$ | |
| 32 | 6-Cl | H | OMe | $CO_2Et$ | |
| 33 | 6-Cl | H | NHMe | $CO_2Et$ | |
| 34 | 6-Cl | H | $NMe_2$ | $CO_2Et$ | |
| 35 | 6-Cl | H | $CONMe_2$ | $CO_2Et$ | |
| 36 | 6-Cl | H | $CO_2Et$ | $CO_2Et$ | |
| 37 | 6-Cl | H | CN | $CO_2Pr^n$ | 81~82 |
| 38 | 6-Cl | H | CN | $CO_2Pr^i$ | 101~103 |
| 39 | 6-Cl | H | CN | $CO_2Bu^n$ | 73~75 |
| 40 | 6-Cl | H | CN | $CO_2CH_2CH_2Cl$ | 114~116.5 |

Table 3 (continued)

| Compound No. | $X_1$ | $X_2$ | Y | Q | m.p.(℃) |
|---|---|---|---|---|---|
| 41 | 6-Cl | H | CN | $CO_2CH_2CH=CH_2$ | 78~79 |
| 42 | 6-Cl | H | CN | $CO_2CH_2C\equiv CH$ | 127~129 |
| 43 | 6-Cl | H | CN | $CO_2CH_2CH_2SiMe_3$ | 90~91 |
| 44 | 6-Cl | H | CN | $CO_2Bu^t$ | 110~112 |
| 45 | 6-Cl | H | CN | 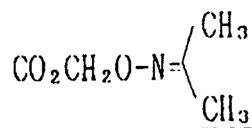 | |
| 46 | 6-Cl | H | CN | 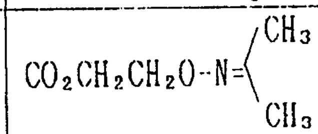 | 77~79 |
| 47 | 6-Cl | H | CN | CONHPh | 184.5~186 |
| 48 | 6-Cl | H | CN | CONHMe | |
| 49 | 6-Cl | H | CN | $CONMe_2$ | |
| 50 | 6-Cl | H | CN | CN | |
| 51 | 6-Cl | H | CN | COSMe | |
| 52 | 6-Cl | H | CN | $CO_2CH_2CO_2Me$ | |
| 53 | 6-Cl | H | CN | $CO_2CH(OH)CH_2CO_2Me$ | |
| 54 | 6-Cl | H | $NO_2$ | $CO_2H$ | |

Table 3 (continued)

| Compound No. | $X_1$ | $X_2$ | Y | Q | m.p.(℃) |
|---|---|---|---|---|---|
| 55 | 6-Cl | H | $NO_2$ | $CO_2Me$ | |
| 56 | 6-Cl | H | $NO_2$ | $CO_2Pr^n$ | |
| 57 | 6 Cl | H | $NO_2$ | $CO_2Pr^i$ | |
| 58 | 6-Cl | H | $NO_2$ | $CO_2Bu^n$ | |
| 59 | 6-Cl | H | $NO_2$ | $CO_2Bu^t$ | |
| 60 | 6-Cl | H | $NO_2$ | $CO_2CH_2CH_2Cl$ | |
| 61 | 6-Cl | H | $NO_2$ | $CO_2CH_2CH=CH_2$ | |
| 62 | 6-Cl | H | $NO_2$ | $CO_2CH_2C \equiv CH$ | |
| 63 | 6-Cl | H | $NO_2$ | $CO_2CH_2CH_2SiMe_3$ | |
| 64 | 6-Cl | H | $NO_2$ | $CH_3$<br>$CO_2CH_2O-N=$<br>$CH_3$ | |
| 65 | 6-Cl | H | $NO_2$ | $CH_3$<br>$CO_2CH_2CH_2O-N=$<br>$CH_3$ | |
| 66 | 6-Cl | H | $NO_2$ | CONHPh | |
| 67 | 6-Cl | H | $NO_2$ | CONMePh | |
| 68 | 6-Cl | H | $NO_2$ | CONHMe | |

Table 3 (continued)

| Compound No. | $X_1$ | $X_2$ | Y | Q | m.p.(℃) |
|---|---|---|---|---|---|
| 69 | 6-Cl | H | $NO_2$ | $CONMe_2$ | |
| 70 | 6-Cl | H | $NO_2$ | CN | |
| 71 | 6-Cl | H | $NO_2$ | COSMe | |
| 72 | 6-Cl | H | $NO_2$ | $CO_2CH_2CO_2Me$ | |
| 73 | 6-Cl | H | $NO_2$ | $CO_2CH(OH)CH_2CO_2Me$ | |
| 74 | 6-F | H | H | $CO_2Et$ | |
| 75 | 6-F | H | F | $CO_2Et$ | |
| 76 | 6-F | H | Cl | $CO_2Et$ | |
| 77 | 6-F | H | Br | $CO_2Et$ | |
| 78 | 6-F | H | $COCH_3$ | $CO_2Et$ | |
| 79 | 6-F | H | CHO | $CO_2Et$ | |
| 80 | 6-F | H | SMe | $CO_2Et$ | |
| 81 | 6-F | H | SOMe | $CO_2Et$ | |
| 82 | 6-F | H | $SO_2Me$ | $CO_2Et$ | |
| 83 | 6-F | H | $CH_3$ | $CO_2Et$ | |
| 84 | 6-F | H | $CF_3$ | $CO_2Et$ | |

Table 3 (continued)

| Compound No. | $X_1$ | $X_2$ | Y | Q | m.p. (°C) |
|---|---|---|---|---|---|
| 85 | 6-F | H | $SO_2NMe_2$ | $CO_2Et$ | |
| 86 | 6-F | H | OMe | $CO_2Et$ | |
| 87 | 6-F | H | NHMe | $CO_2Et$ | |
| 88 | 6-F | H | $NMe_2$ | $CO_2Et$ | |
| 89 | 6-F | H | $CONMe_2$ | $CO_2Et$ | |
| 90 | 6-F | H | $CO_2Et$ | $CO_2Et$ | |
| 91 | 6-F | H | CN | $CO_2H$ | |
| 92 | 6-F | H | CN | $CO_2Me$ | |
| 93 | 6-F | H | CN | $CO_2Et$ | |
| 94 | 6-F | H | CN | $CO_2Pr^n$ | |
| 95 | 6-F | H | CN | $CO_2Pr^i$ | |
| 96 | 6-F | H | CN | $CO_2Bu^n$ | |
| 97 | 6-F | H | CN | $CO_2Bu^t$ | |
| 98 | 6-F | H | CN | $CO_2CH_2CH_2Cl$ | |
| 99 | 6-F | H | CN | $CO_2CH_2CH=CH_2$ | |
| 100 | 6-F | H | CN | $CO_2CH_2C \equiv CH$ | |

Table 3 (continued)

| Compound No. | $X_1$ | $X_2$ | Y | Q | m.p.(℃) |
|---|---|---|---|---|---|
| 101 | 6-F | H | CN | $CO_2CH_2CH_2SiMe_3$ | |
| 102 | 6-F | H | CN | 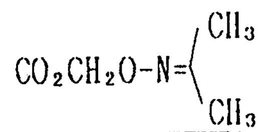 | |
| 103 | 6-F | H | CN | 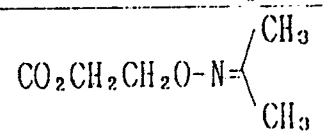 | |
| 104 | 6-F | H | CN | CONHPh | |
| 105 | 6-F | H | CN | CONMePh | |
| 106 | 6-F | H | CN | CONHMe | |
| 107 | 6-F | H | CN | $CONMe_2$ | |
| 108 | 6-F | H | CN | CN | |
| 109 | 6-F | H | CN | COSMe | |
| 110 | 6-F | H | CN | $CO_2CH_2CO_2Me$ | |
| 111 | 6-F | H | CN | $CO_2CH(OH)CH_2CO_2Me$ | |
| 112 | 6-F | H | $NO_2$ | $CO_2H$ | |
| 113 | 6-F | H | $NO_2$ | $CO_2Me$ | |
| 114 | 6-F | H | $NO_2$ | $CO_2Et$ | |

Table 3 (continued)

| Compound No. | $X_1$ | $X_2$ | Y | Q | m.p.(℃) |
|---|---|---|---|---|---|
| 115 | 6-F | H | $NO_2$ | $CO_2Pr^n$ | |
| 116 | 6-F | H | $NO_2$ | $CO_2Pr^i$ | |
| 117 | 6-F | H | $NO_2$ | $CO_2Bu^n$ | |
| 118 | 6-F | H | $NO_2$ | $CO_2Bu^t$ | |
| 119 | 6-F | H | $NO_2$ | $CO_2CH_2CH_2Cl$ | |
| 120 | 6-F | H | $NO_2$ | $CO_2CH_2CH=CH_2$ | |
| 121 | 6-F | H | $NO_2$ | $CO_2CH_2C≡CH$ | |
| 122 | 6-F | H | $NO_2$ | $CO_2CH_2CH_2SiMe_3$ | |
| 123 | 6-F | H | $NO_2$ | 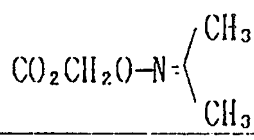 | |
| 124 | 6-F | H | $NO_2$ | 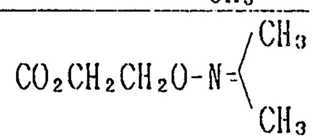 | |
| 125 | 6-F | H | $NO_2$ | CONHPh | |
| 126 | 6-F | H | $NO_2$ | CONMePh | |
| 127 | 6-F | H | $NO_2$ | CONHMe | |
| 128 | 6-F | H | $NO_2$ | $CONMe_2$ | |

Table 3 (continued)

| Compound No. | $X_1$ | $X_2$ | Y | Q | m.p.(℃) |
|---|---|---|---|---|---|
| 129 | 6-F | H | $NO_2$ | CN | |
| 130 | 6-F | H | $NO_2$ | COSMe | |
| 131 | 6-F | H | $NO_2$ | $CO_2CH_2CO_2Me$ | |
| 132 | 6-F | H | $NO_2$ | $CO_2CH(OH)CH_2CO_2Me$ | |
| 133 | 6-Br | H | H | $CO_2Et$ | |
| 134 | 6-Br | H | F | $CO_2Et$ | |
| 135 | 6-Br | H | Cl | $CO_2Et$ | |
| 136 | 6-Br | H | Br | $CO_2Et$ | |
| 137 | 6-Br | H | $COCH_3$ | $CO_2Et$ | |
| 138 | 6-Br | H | CHO | $CO_2Et$ | |
| 139 | 6-Br | H | SMe | $CO_2Et$ | |
| 140 | 6-Br | H | SOMe | $CO_2Et$ | |
| 141 | 6-Br | H | $SO_2Me$ | $CO_2Et$ | |
| 142 | 6-Br | H | $CH_3$ | $CO_2Et$ | |
| 143 | 6-Br | H | $CF_3$ | $CO_2Et$ | |
| 144 | 6-Br | H | $SO_2NMe_2$ | $CO_2Et$ | |

Table 3 (continued)

| Compound No. | $X_1$ | $X_2$ | Y | Q | m.p.(℃) |
|---|---|---|---|---|---|
| 145 | 6-Br | H | OMe | $CO_2Et$ | |
| 146 | 6-Br | H | NHMe | $CO_2Et$ | |
| 147 | 6-Br | H | $NMe_2$ | $CO_2Et$ | |
| 148 | 6-Br | H | $CONMe_2$ | $CO_2Et$ | |
| 149 | 6-Br | H | $CO_2Et$ | $CO_2Et$ | |
| 150 | 6-Br | H | CN | $CO_2H$ | |
| 151 | 6-Br | H | CN | $CO_2Me$ | |
| 152 | 6-Br | H | CN | $CO_2Et$ | 82～84 |
| 153 | 6-Br | H | CN | $CO_2Pr^n$ | |
| 154 | 6-Br | H | CN | $CO_2Pr^i$ | |
| 155 | 6-Br | H | CN | $CO_2Bu^n$ | |
| 156 | 6-Br | H | CN | $CO_2Bu^t$ | |
| 157 | 6-Br | H | CN | $CO_2CH_2CH_2Cl$ | |
| 158 | 6-Br | H | CN | $CO_2CH_2CH{=}CH_2$ | |
| 159 | 6-Br | H | CN | $CO_2CH_2C{\equiv}CH$ | |
| 160 | 6-Br | H | CN | $CO_2CH_2CH_2SiMe_3$ | |

Table 3 (continued)

| Compound No. | $X_1$ | $X_2$ | Y | Q | m.p.(℃) |
|---|---|---|---|---|---|
| 161 | 6-Br | H | CN | 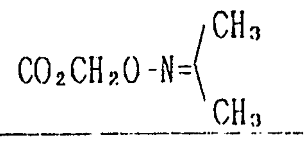 | |
| 162 | 6-Br | H | CN | 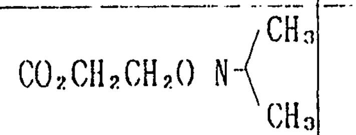 | |
| 163 | 6-Br | H | CN | CONHPh | |
| 164 | 6-Br | H | CN | CONMePh | |
| 165 | 6-Br | H | CN | CONHMe | |
| 166 | 6-Br | H | CN | $CONMe_2$ | |
| 167 | 6-Br | H | CN | CN | |
| 168 | 6-Br | H | CN | COSMe | |
| 169 | 6-Br | H | CN | $CO_2CH_2CO_2Me$ | |
| 170 | 6-Br | H | CN | $CO_2CH(OH)CH_2CO_2Me$ | |
| 171 | 6-Br | H | $NO_2$ | $CO_2H$ | |
| 172 | 6-Br | H | $NO_2$ | $CO_2Me$ | |
| 173 | 6-Br | H | $NO_2$ | $CO_2Et$ | |
| 174 | 6-Br | H | $NO_2$ | $CO_2Pr^n$ | |

Table 3 (continued)

| Compound No. | $X_1$ | $X_2$ | Y | Q | m.p.(℃) |
|---|---|---|---|---|---|
| 175 | 6-Br | H | $NO_2$ | $CO_2Pr^i$ | |
| 176 | 6-Br | H | $NO_2$ | $CO_2Bu^n$ | |
| 177 | 6-Br | H | $NO_2$ | $CO_2Bu^t$ | |
| 178 | 6-Br | H | $NO_2$ | $CO_2CH_2CH_2Cl$ | |
| 179 | 6-Br | H | $NO_2$ | $CO_2CH_2C{=}CH_2$ | |
| 180 | 6-Br | H | $NO_2$ | $CO_2CH_2C{\equiv}CH$ | |
| 181 | 6-Br | H | $NO_2$ | $CO_2CH_2CH_2SiMe_3$ | |
| 182 | 6-Br | H | $NO_2$ | $CO_2CH_2O{-}N{=}C(CH_3)_2$ | |
| 183 | 6-Br | H | $NO_2$ | $CO_2CH_2CH_2O{-}N{=}C(CH_3)_2$ | |
| 184 | 6-Br | H | $NO_2$ | CONHPh | |
| 185 | 6-Br | H | $NO_2$ | CONMePh | |
| 186 | 6-Br | H | $NO_2$ | CONHMe | |
| 187 | 6-Br | H | $NO_2$ | $CONMe_2$ | |
| 188 | 6-Br | H | $NO_2$ | CN | |

Table 3 (continued)

| Compound No. | $X_1$ | $X_2$ | Y | Q | m.p.(℃) |
|---|---|---|---|---|---|
| 189 | 6-Br | H | $NO_2$ | COSMe | |
| 190 | 6-Br | H | $NO_2$ | $CO_2CH_2CO_2Me$ | |
| 191 | 6-Br | H | $NO_2$ | $CO_2CH(OH)CH_2CO_2Me$ | |
| 192 | 6-Me | H | CN | $CO_2Et$ | 127~129 |
| 193 | 6-Me | H | $NO_2$ | $CO_2Et$ | |
| 194 | 5-Me | H | CN | $CO_2Et$ | |
| 195 | 5-Me | H | $NO_2$ | $CO_2Et$ | |
| 196 | 7-Me | H | CN | $CO_2Et$ | 138~139 |
| 197 | 7-Me | H | $NO_2$ | $CO_2Et$ | |
| 198 | 8-Me | H | CN | $CO_2Et$ | 101~102 |
| 199 | 8-Me | H | $NO_2$ | $CO_2Et$ | |
| 200 | 5-Cl | H | CN | $CO_2Et$ | |
| 201 | 5-Cl | H | $NO_2$ | $CO_2Et$ | |
| 202 | 7-Cl | H | CN | $CO_2Et$ | |
| 203 | 7-Cl | H | $NO_2$ | $CO_2Et$ | |
| 204 | 8-Cl | H | CN | $CO_2Et$ | |

Table 3 (continued)

| Compound No. | $X_1$ | $X_2$ | Y | Q | m.p.(℃) |
|---|---|---|---|---|---|
| 205 | 8-Cl | H | $NO_2$ | $CO_2Et$ | |
| 206 | 6-$CF_3$ | H | CN | $CO_2Et$ | 86~87 |
| 207 | 6-$CF_3$ | H | $NO_2$ | $CO_2Et$ | |
| 208 | 6-Cl | 7-Cl | CN | $CO_2Et$ | 117~119 |
| 209 | 6-Cl | 7-Cl | $NO_2$ | $CO_2Et$ | |
| 210 | 6-$NO_2$ | H | CN | $CO_2Et$ | |
| 211 | 6-$NO_2$ | H | $NO_2$ | $CO_2Et$ | |
| 212 | 6-Cl | H | CN | CO- 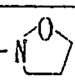 | |
| 213 | 6-Cl | H | CN | COSEt | 104~105.5 |
| 214 | 6-Cl | H | CN | $CONEt_2$ | oil |
| 215 | 6-Cl | H | CN | CO- 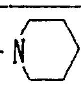 | 113~114 |
| 216 | 6-Cl | H | CN | $CO_2CH_2$- 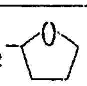 | 89.5~91 |
| 217 | 6-Cl | H | CN | $CO_2(CH_2)_5CH_3$ | 68.5~69.5 |
| 218 | 6-Cl | H | CN | $CO_2$- 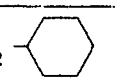 | 94~96 |
| 219 | 6-Cl | H | CN | $CO_2Ph$ | 117~118 |
| 220 | 6-Cl | H | CN | $CO_2CH_2Ph$ | 107~109 |

| Formulation Example 1 | |
|---|---|
| Emulsion | |
| Compound No. 8 | 20 weight % |
| Xylene | 74 weight % |
| Sodium dodecylbenzenesulfonate (DBS-50X®) | 3 weight % |
| Polyethyleneglycol ether (Nonipole 85®) | 3 weight % |

These components were mixed to give an emulsion which can be used by appropriately diluting with water.

| Formulation Example 2 | |
|---|---|
| Wettable powders | |
| Compound No. 41 | 41 weight % |
| Sodium ligninsulfonate | 5 weight % |
| Polyethyleneglycol ether (Nonipole 85®) | 5 weight % |
| Clay | 50 weight % |

These compounds were mixed and crushed to give the captioned formulation, which can be used by appropriately diluting with water.

| Formulation Example 3 | |
|---|---|
| Granules | |
| Compound No. 47 | 2.5 weight % |
| Sodium ligninsulfonate | 5 weight % |
| CMC (Cerogen 7A®) | 3 weight % |
| Clay | 89.5 weight % |

Water was added to a mixture of these components and granulated by a conventional method.

Test 1

Herbicidal effect by treatment after germination in upland field

Clay loam was filled in Diphy-pot® of 10cm diameter, and crabgrass, foxtail, corn and soybean were seeded in each of the pots. After covering up the seeds with soil (0.5cm), cultivation was continued in a greenhouse for 2 - 3 weeks. At the 2nd - 3rd leaf stage, diluted solution of the compound (I) was sprayed on stem and leaves by a spray gun at the rate of 20g, 10g or 5g per 1 are. The diluted solution was prepared by dissolving the compound (I) (20g, 10g or 5g) in acetone (500ml) containing a surface active agent, Tween 20® (2% W/V), and then diluting with water to a total volume of 5l.

Herbicidal effect and damage against crops of each test compound were evaluated 2 weeks after the treatment in accordance with the following index.

| Index No. | Effect | Inhibition rate % (herbicidal rate) |
|---|---|---|
| 0 | no | 0 |
| 1 | faint | 0.1 - 50.0 |
| 2 | weak | 50.1 - 75.0 |
| 3 | middle | 75.1 - 87.5 |
| 4 | strong | 87.6 - 99.9 |
| 5 | excellent | 100 |

Damage against soybean is indicated in accordance with the following index (same in the following tests).

| Index No. | Effect | Inhibition rate % (herbicidal rate) |
|---|---|---|
| 0 | no | 0 |
| 1 | faint | 0.1 - 12.5 |
| 2 | light | 12.6 - 25.0 |
| 3 | middle | 25.1 - 50.0 |
| 4 | bad | 50.1 - 99.9 |
| 5 | heavy | 100 |

The test results are shown in the following Table

Table 4

| Compound No. | Amount (g/a) | Damage Soybean | Herbicidal Effect | | |
|---|---|---|---|---|---|
| | | | Crabgrass | Foxtail | Corn |
| No. 8 | 20 | 0 | 5 | 5 | 5 |
| | 10 | 0 | 5 | 5 | 5 |
| | 5 | 0 | 5 | 5 | 5 |
| No. 9 | 20 | 0 | 5 | 5 | 5 |
| | 10 | 0 | 4 | 5 | 5 |
| | 5 | 0 | 4 | 4 | 5 |
| No. 10 | 20 | 0 | 4 | 4 | 5 |
| | 10 | 0 | 4 | 4 | 5 |
| | 5 | 0 | 3 | 3 | 5 |
| No. 37 | 20 | 0 | 5 | 5 | 5 |
| | 10 | 0 | 5 | 5 | 5 |
| | 5 | 0 | 5 | 4 | 5 |
| No. 38 | 20 | 0 | 5 | 5 | 5 |
| | 10 | 0 | 5 | 5 | 5 |
| | 5 | 0 | 4 | 4 | 5 |
| No. 39 | 20 | 0 | 4 | 5 | 5 |
| | 10 | 0 | 4 | 4 | 5 |
| | 5 | 0 | 3 | 4 | 5 |
| No. 41 | 20 | 0 | 5 | 5 | 5 |
| | 10 | 0 | 5 | 5 | 5 |
| | 5 | 0 | 5 | 5 | 5 |

Table 4 (continued)

| Compound No. | Amount (g/a) | Damage Soybean | Herbicidal Effect | | |
|---|---|---|---|---|---|
| | | | Crabgrass | Foxtail | Corn |
| No. 42 | 20 | 0 | 4 | 4 | 5 |
| | 10 | 0 | 3 | 4 | 5 |
| | 5 | 0 | 4 | 4 | 5 |
| No. 152 | 20 | 0 | 5 | 5 | 5 |
| | 10 | 0 | 4 | 5 | 5 |
| | 5 | 0 | 4 | 5 | 5 |
| No. 192 | 20 | 0 | 2 | 4 | 4 |
| | 10 | 0 | 2 | 4 | 4 |
| | 5 | 0 | 1 | 4 | 4 |
| No. 213 | 20 | 0 | 4 | 5 | 5 |
| | 5 | 0 | 4 | 5 | 5 |
| No. 216 | 20 | 0 | 4 | 5 | 5 |
| | 5 | 0 | 4 | 4 | 5 |
| No. 217 | 20 | 0 | 4 | 5 | 5 |
| | 5 | 0 | 5 | 5 | 5 |
| No. 218 | 20 | 0 | 4 | 4 | 5 |
| | 5 | 0 | 4 | 4 | 5 |
| No. 219 | 20. | 0 | 5 | 5 | 5 |
| | 5 | 0 | 4 | 5 | 5 |

Table 4 (continued)

| Compound No. | Amount (g/a) | Damage Soybean | Herbicidal Effect | | |
|---|---|---|---|---|---|
| | | | Crabgrass | Foxtail | Corn |
| No. 220 | 20 | 0 | 5 | 5 | 5 |
| | 5 | 0 | 4 | 4 | 4 |
| No. 221 | 5 | 0 | 5 | 5 | 5 |

Test 2

Comparison with control by treatment after germination in upland field

Clay loam was filled in Diphy-pot® of 10cm diameter, and crabgrass, foxtail, barnyardgrass, fall panicum and Johnsongrass were seeded in each of the pots. After covering up the seeds with soil (0.5cm), cultivation was continued in a greenhouse for 2 - 3 weeks. At the 2nd - 3rd leaf stage, diluted solution of the compound (I) and fixed amount of a control agent, Alloxydim were sprayed on stem and leaves by using a spray gun.

Two weeks after the treatment, herbicidal effect and damage against crops were evaluated by the same method at Test 1. The results are shown in Table 5.

Table 5

| Compound No. | Amount (g/a) | Damage Soybean | Herbicidal Effect | | | | |
|---|---|---|---|---|---|---|---|
| | | | Crabgrass | Foxtail | Barnyard-grass | Fall panicum | Johnson-grass |
| No. 8 | 20 | 0 | 5 | 5 | 5 | 5 | 5 |
| | 10 | 0 | 5 | 5 | 5 | 5 | 5 |
| | 5 | 0 | 5 | 5 | 5 | 5 | 5 |
| | 2.5 | 0 | 5 | 5 | 5 | 5 | 5 |
| No. 152 | 20 | 0 | 5 | 5 | 5 | 5 | 5 |
| | 10 | 0 | 5 | 5 | 5 | 5 | 5 |
| | 5 | 0 | 4 | 4 | 4 | 5 | 5 |
| | 2.5 | 0 | 4 | 4 | 3 | 4 | 5 |
| No. 213 | 20 | 0 | 4 | 5 | - | - | - |
| | 5 | 0 | 4 | 5 | 5 | 4 | 5 |
| | 2.5 | 0 | 4 | 4 | 5 | 4 | 5 |

Table 5 (continued)

| Compound No. | Amount (g/a) | Damage Soybean | Herbicidal Effect Crabgrass | Foxtail | Barnyard-grass | Fall panicum | Johnson-grass |
|---|---|---|---|---|---|---|---|
| No. 216 | 20 | 0 | 4 | 5 | - | - | - |
| | 5 | 0 | 4 | 4 | 5 | 5 | 5 |
| | 2.5 | 0 | 4 | 4 | 5 | 5 | 5 |
| No. 219 | 20 | 0 | 5 | 5 | - | - | - |
| | 5 | 0 | 4 | 5 | 5 | 4 | 5 |
| | 2.5 | 0 | 4 | 5 | 5 | 4 | 5 |
| Alloxydim[1) ] (control) | 10 | 0 | 4 | 4 | 5 | 5 | 1 |
| | 5 | 0 | 1 | 4 | 5 | 5 | 1 |

1) Sodium salt : Chemical Name, sodium 3-(1-allyloxyaminobutyliden)-6,6-dimethyl-2,4-dioxocyclohexanecarboxylate

Test 3

Selectivity test against rice plant

Clay loam of paddy field was filled in 1/10000 are Wagner pot, and barnyard grass (Echinochloa crus-galli; P. Veauv. var. caudata Kitagawa) was seeded. Cultivation at 3cm of flooding was continued for a fixed term.

On the other hand, Clay loam of paddy field was filled in 1/10000 are Wagner pot, and water was poured thereto. After tilling, two stumps of young rice plants at 2nd leaf stage were transplanted, and the pot was flooded to 3cm deep. 7 Days after transplantation, at 1st leaf stage of the barnyard grass, diluted solution containing a fixed amount of the compound (I) was applied to the pot.

The diluted solution was prepared by dissolving a fixed amount of the test compound (I) in acetone (2 liter) containing a surface active agent, Tween 20® (2% W/V) and then diluting with water to a total volume 50 liter.

Three weeks after the treatment, herbicidal effects against each kind of weeds and damage against transplanted rice plants were evaluated by the same method as Test 1. The results are shown in the following Table 6.

Table 6

| Compound No. | Amount (g/a) | Damage rice plants | Herbicidal Effect (Barnyard grass) |
|---|---|---|---|
| No. 5 | 20 | 0 | 5 |
| | 10 | 0 | 3 |
| | 5 | 0 | 5 |
| No. 8 | 20 | 2 | 5 |
| | 10 | 1 | 5 |
| | 5 | 0 | 5 |
| | 2.5 | 0 | 5 |
| No. 47 | 20 | 0 | 5 |
| | 10 | 0 | 5 |
| | 5 | 0 | 5 |
| No. 152 | 20 | 2 | 5 |
| | 10 | 2 | 5 |
| | 5 | 1 | 4 |
| No. 192 | 20 | 0 | 5 |
| | 10 | 0 | 4 |
| | 5 | 0 | 4 |
| No. 213 | 10 | 1 | 5 |
| | 5 | 1 | 5 |
| | 2.5 | 1 | 5 |

Table 6 (continued)

| Compound No. | Amount (g/a) | Damage rice plants | Herbicidal Effect (Barnyard grass) |
|---|---|---|---|
| No. 215 | 20 | 0 | 5 |
| | 10 | 0 | 5 |
| | 5 | 0 | 4 |
| No. 44 | 20 | 1 | 5 |
| | 10 | 0 | 4 |
| | 5 | 0 | 4 |
| Fluazifop[2)] control | 10 | 3 | 5 |
| | 5 | 4 | 5 |
| | 2.5 | 4 | 5 |

2) n-Butyl 2-[4-(5-trifluoromethyl-2-pyridyloxy) phenoxy]propionate

## Claims

1. A compound of the formula (I):

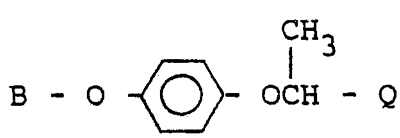

(I)

in which B is an imidazo[1,2-a]pyridin-2-yl group which may be substituted, Q is a cyano group or a group of the formula:

$$-\overset{\overset{O}{\|}}{C}-A-R$$

wherein A is an oxygen or sulfur atom or a group of the formula :-$NR^1$- (wherein $R^1$ is a hydrogen atom or a lower alkyl group), R is a hydrogen atom, a hydrocarbon group which may be substituted or a heterocyclic group which may be substituted; when A is -$NR^1$-, R and $R^1$ may combine to form a heterocyclic group together with the adjacent nitrogen atom; or when A is an oxygen atom or -$NR^1$-, R may be a group of the formula:

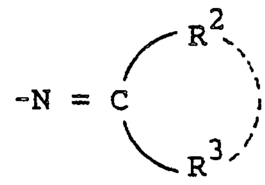

wherein $R^2$ and $R^3$ each are a lower alkyl, lower alkoxy or lower alkylthio group, or $R^2$ and $R^3$ may form a 5- or 6-membered ring together with the adjacent carbon atom, or a salt thereof.

2. A compound of claim 1 in which the substituent(s) on the pyridine ring in the imidazo[1,2-a]pyridin-2-yl group which may be substituted is one or two and is a halogen atom, nitro group or a lower alkyl group which may be substituted by halogen(s), and the substituent on the imidazole ring in the imidazo[1,2-a]-pyridin-2-yl group which may be substituted is a halogen atom, cyano group, nitro group, a $C_{1-7}$ acyl group, a lower alkoxycarbonyl group, a carbamoyl group which may be substituted by a lower alkyl, carboxyl group, a lower alkylthio group, a lower alkylsulfinyl group, a lower alkylsulfonyl group, a lower alkylamino, a di-lower alkylamino, a lower alkoxy, a lower alkylsulfamoyl, a di-lower alkylsulfamoyl, or a lower alkyl group which may be substituted by one to four of a halogen, hydroxyl, amino, a lower alkylamino, a di-lower alkylamino, a lower alkoxy and a lower alkylthio.

3. A compound of claim 1 in which in the group of $-\overset{O}{\overset{\|}{C}}-A-R$, A is an oxygen atom and R is a hydrocarbon group which may be substituted.

4. A compound of claim 3 in which the hydrocarbon group is a lower alkyl, lower alkenyl or alkynyl group.

5. A compound of claim 1 which is a compound of the formula:

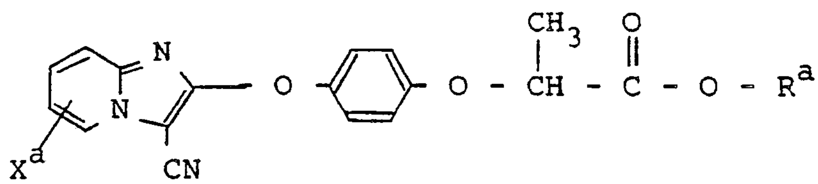 $(I^a)$

wherein $X^a$ is a halogen atom, and $R^a$ is a $C_{1-4}$ alkyl or $C_{2-4}$ alkenyl group, or its salt.

6. A compound of claim 1 which is a compound of the formula:

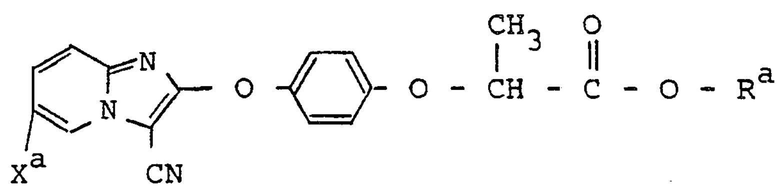 $(I^b)$

wherein the symbols have the same meanings as defined above in claim 5, or its salt.

7. A compound of claim 1 which is the compound of the formula:

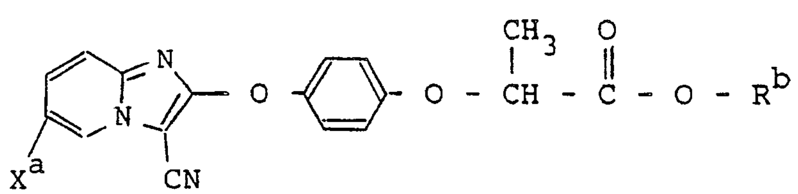 $(I^c)$

wherein $X^a$ has the same meaning as defined above in claim 5 and $R^b$ is a $C_{2-4}$ alkenyl group, or its salt.

8. A compound of claim 6 in which $X^a$ is chlorine, bromine or fluorine atom and $R^a$ is methyl, ethyl, n-propyl, i-propyl, n-butyl, i-butyl or t-butyl group.

9. A compound of claim 7 in which $X^a$ is chlorine, bromine or fluorine atom and $R^a$ is allyl group.

10. A compound of claim 1 which is in the form of optical isomers or a mixture thereof.

11. A compound of claim 1, which is n-propyl 2-[4-(3-cyano-6-chloroimidazo[1,2-a]pyridin-3-yloxy)-phenoxy]propionate.

12. A compound of claim 1, which is iso-propyl 2-[4-(3-cyano-6-chloroimidazo[1,2-a]pyridin-2-yloxy)-phenoxy]propionate.

13. A compound of claim 1, which is n-butyl 2-[4-(3-cyano-6-chloroimidazo[1,2-a]pyridin-2-yloxy)-phenoxy]propionate.

14. A compound of claim 1, which is allyl 2-[4-(3-cyano-6-chloroimidazo[1,2-a]pyridin-2-yloxy)phenoxy]-propionate.

15. A compound of claim 1, which is ethyl R-(+)-2-[4-(3-cyano-6-chloroimidazo[1,2-a]pyridin-2-yloxy)-phenoxy]propionate.

16. A process for preparing a compound of the formula (I) in claim 1 or its salt, which comprises reacting a compound of the formula (II):

B - Z (II)

wherein Z is a removable group, and B is as defined in claim 1,
or its salt with a compound of the formula (III):

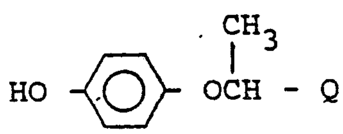

(III)

wherein Q is as defined in claim 1,
in the presence of a base.

17. A process for preparing a compound of the formula (I) in claim 1 or its salt which comprises reacting a compound of the formula (IV):

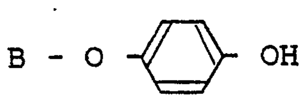

(IV)

wherein B is as defined in claim 1,
or its salt with a compound of the formula (V):

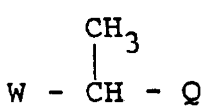

(V)

wherein W is a removable group, and Q is as defined in claim 1,
in the presence of a base.

18. A process of claim 16 or 17 in which the base is an inorganic base.

19. A process of claim 16 or 17 in which the removable group for the symbol Z or W is a halogen atom, a $C_{6-10}$ aromatic hydrocarbon sulfonyloxy group or a $C_{1-6}$ alkane sulfonyloxy group.

20. A process of claim 16 or 17 which is adopted to prepare a compound as claimed in any one of claims 2 -15 or its salt.

21. A herbicidal composition which comprises a compound of the formula (I) in claim 1 or its salt and a diluent or carrier.

22. An use of a compound of the formula (I) in claim 1 or its salt in the preparation of a herbicidal composition.